(19) Europäisches Patentamt
European Patent Office
Office européen des brevets

(11) Publication number: **0 257 419 A1**

(12) **EUROPEAN PATENT APPLICATION**

(21) Application number: **87111529.1**

(22) Date of filing: **10.08.87**

(51) Int. Cl.⁴: **C 07 D 499/00**
// C07F7/18, C07D205/08

(30) Priority: **12.08.86 GB 8619605**

(43) Date of publication of application: **02.03.88 Bulletin 88/9**

(84) Designated Contracting States: **AT BE CH DE ES FR GB GR IT LI LU NL SE**

(71) Applicant: **HOECHST AKTIENGESELLSCHAFT, Postfach 80 03 20, D-6230 Frankfurt am Main 80 (DE)**

(72) Inventor: **Barker, Andrew John, 152 Sutherland Grove, West Bletchley Milton Keynes Bucks (GB)**
Inventor: **Cooke, Michael David, 10 Westbury lane, Newport Pagnell Bucks (GB)**

(54) **Process for the preparation of antibacterial 7-oxo-4-thia-1-azabicyclo[3.2.0]hept-2-ene derivatives.**

(57) Compounds Ia

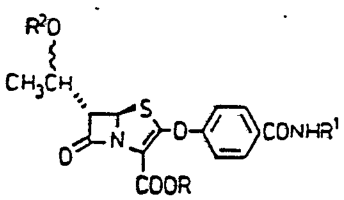

(Ia)

with
R = H, protecting group
$R^1$ = H, alkyl
$R^2$ = H, protecting group or salts thereof are prepared by
(1) producing compound Ib

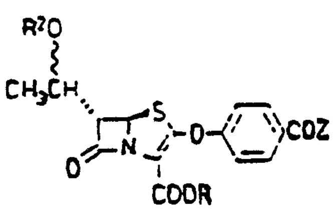

(Ib)

with
Z = $-NHR^3$, $-NR^3R^3$, $-NR^1R^3$, $-R^4$, OH
$R^3$ = protecting group
$R^4$ = carboxyl activating group or –COZ being –CN by
(A) heating II

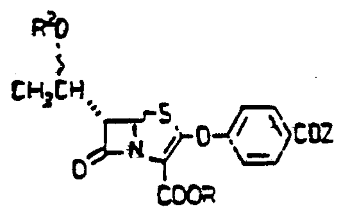

(II)

(B) reacting IV

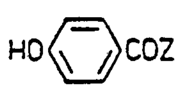

(IV)

EP 0 257 419 A1

with III

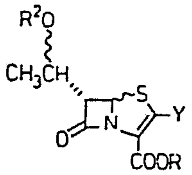

(III)

y being a leaving group

(C) cyclising Va, Vb or Vc

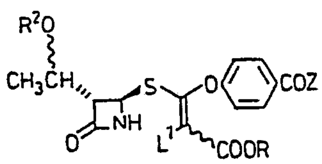

(Va)

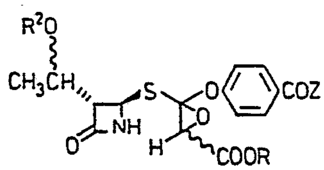

(Vb)

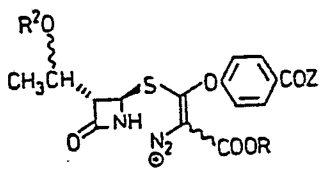

(Vc)

($L^1$ = Cl, Br)

(D) cyclising VIa, VIb, VIc, VId

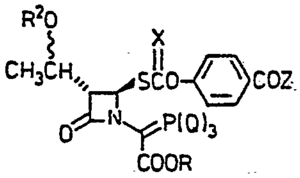

(VIa)

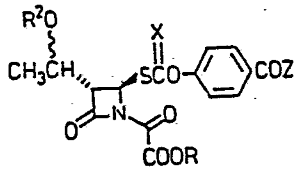

(VIb)

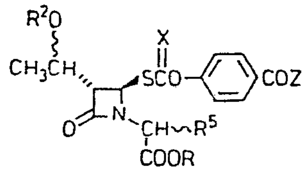

(VIc)

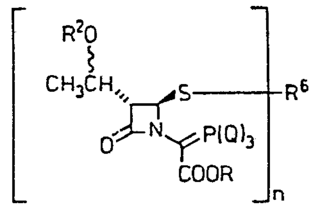

(VId)

X = O, S
$R^5$ = Cl, Br
$R^6$ = Cu(II), P(II) or Hg(II)

(E) cyclising VIIIa or VIIIb

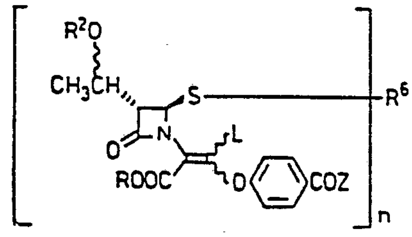

(VIIIa)

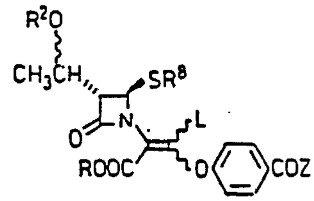

(VIIIb)

L being a leaving group and

(2) removing protecting groups, reacting COZ groups with ammonia or methylamine or converting COZ or CN groups into $CONHR^1$,

(3) converting a compound Ia into another compound Ia.

These penem compounds have antibacterial properties.

# PROCESS FOR THE PREPARATION OF ANTIBACTERIAL 7-OXO-4-THIA-1-AZABICYCLO[3.2.0]HEPT-2-ENE DERIVATIVES

This invention relates to a process for the production of penem derivatives having antibacterial activity and/or β-lactamase inhibitory and/or inactivating activity.

7-Oxo-4-thia-1-azabicyclo[3.2.0]hept-2-ene has the following formula A, and derivatives thereof having an aliphatic side chain at position 6 are numbered as shown in formula B:

(A) 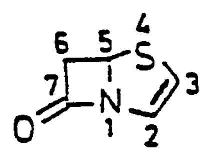 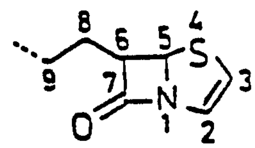 (B)

By an alternative system of nomenclature, the above nucleus A may be described as a "penem", in which case the ring numbering is as shown in formula C, with derivatives having an aliphatic side chain at position 6 being numbered as shown in formula D:

(C) 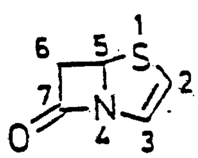 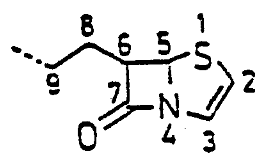 (D)

The present invention relates to 5R,3-(4-aminocarbonylphenoxy)-6S-(1R-hydroxyethyl)-7-oxo-4-thia-1-azabicyclo-[3.2.0]hept-2-ene-2-carboxylic acid and to 5R,3-(4-lower alkylaminocarbonylphenoxy)-6S-(1R-hydroxy)-7-oxo-4-thia-1-azabicyclo[3.2.0]hept-2-ene-2-carboxylic acid, to esters thereof at the 2-carboxylic acid group and/or at the 8-hydroxy group, and to salts thereof, especially

physiologically tolerable salts. The free acids have the formula I given below:

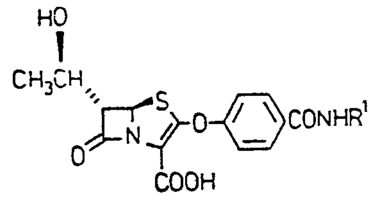

(I)

in which $R^1$ represents a hydrogen atom or an alkyl group having from 1 to 4 carbon atoms. $R^1$ most preferably represents a hydrogen atom or a methyl group.

The compounds of formula I, and salts and esters thereof, are described and claimed in our Application GB 2 161 161 A. These compounds have particularly good activity against a variety of microorganisms, particularly against β-lactamase-producing organisms, including gram positive, gram negative and anaerobic organisms. Moreover, they are more effective than other penems against methicillin-resistant organisms.

Application GB 2 161 161 A discloses certain processes for the production of a compound of formula I and of salts and esters thereof. The present invention relates to further processes for the production of a compound of formula I and of salts and esters thereof. These processes have certain advantages over those disclosed previously. The particular advantages will be given in each case.

Accordingly, the present invention provides a process for the production of a compound of formula Ia

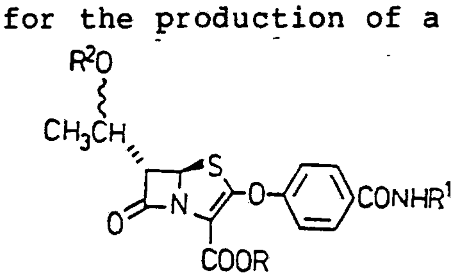

(Ia)

in which R represents a hydrogen atom or a carboxy

protecting group, or an esterifying group that is removable under physiological conditions;

$R^1$ is as defined above, and

$R^2$ represents a hydrogen atom, a hydroxy protecting group, or an esterifying group that is removable under physiological conditions,

or a salt thereof, which comprises

(1) producing a compound of formula Ib

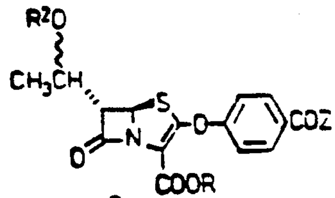

(Ib)

in which R and $R^2$ are as defined above, and

Z represents an -NHR$^3$, -NR$^3$R$^3$, -NR$^1$R$^3$ -R$^4$ or -OH group in which

$R^3$ represents a nitrogen protecting group (in the case of -NR$^3$R$^3$ the two groups $R^3$ may be the same or different), and

$R^4$ represents a carboxylic acid activating group, or the group $XR^{5'}$ with X being oxygen or sulphur and $R^{5'}$ being phenyl, substituted by 1 - 5 substituents selected from the group consisting of CN, $CH_3O$, fluorine, chlorine, or

-COZ represents -CN, by

(A) heating a compound of formula II

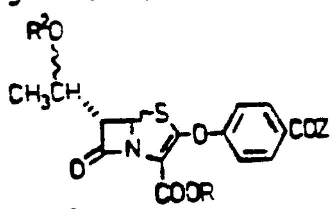

(II)

in which R, $R^2$ and Z are as defined above, or

(B) reacting a compound of formula IV

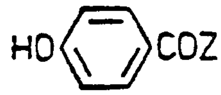

(IV)

in which Z is as defined above, with a compound of formula III

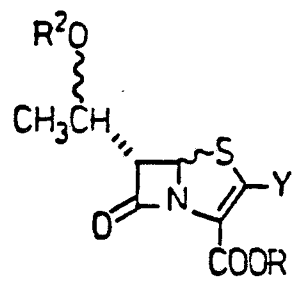

(III)

in which R and $R^2$ are defined as above, and

Y represents a leaving group selected from groups of formula

$$-X\overset{O}{\overset{\parallel}{P}}(OR_a)_2 \quad \text{or} \quad -X\overset{O}{\overset{\parallel}{P}}(R_a)_2$$

in which X represents an oxygen or sulphur atom and the two groups $R_a$, which may be the same or different, each represents a straight or branched chain alkyl group having from 1 to 6 carbon atoms, or a phenyl group which may be unsubstituted or substituted, for example, by one or more substituents selected from halogen atoms, cyano, nitro and methyl groups, especially by a methyl group in the para-position; groups $-OSO_2R_b$ in which $R_b$ represents a straight or branched chain alkyl group having from 1 to 4 carbon atoms, which may be substituted by one or more fluorine atoms, for example, may be fully fluorinated, for example, a $-CF_3$ or $-C_4F_9$ group, or $R_b$ represents a phenyl group that is unsubstituted or is

substituted by a p-nitro, p-bromo- or p-methyl group;

groups $-\overset{O \atop \uparrow}{S}R_a$ and $-SO_2R_a$ in which $R_a$ is defined as above; and groups

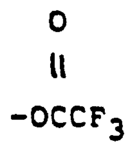

and, if the resulting compound has the formula II

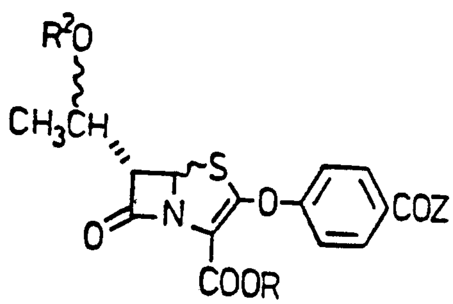

(II)

in which R, $R^2$ and Z are defined as above, converting compound II to compound Ib and/or isolating compound Ib from compound II;

(C) cyclising a compound of formula Va, Vb or Vc

(Va)

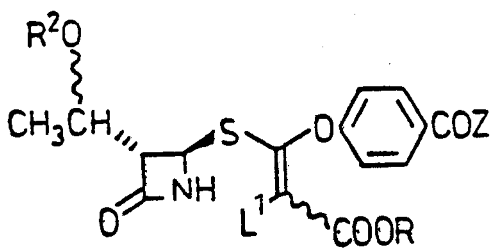

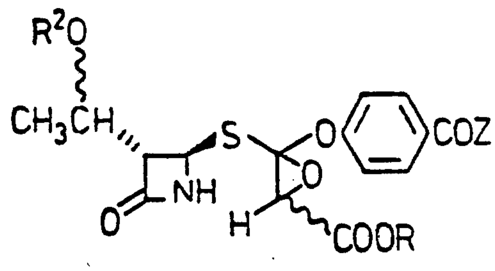

(Vb)

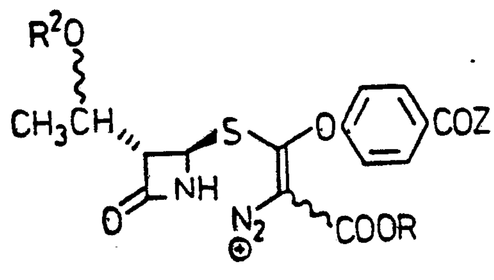

(Vc)

in which R, $R^2$ and Z are as defined above, and $L^1$ represents a chlorine or bromine atom,

(i) in the case of compound Va eliminating $HL^1$

(a) by treatment with a base and/or a metal,

(b) by electrolysis,

(c) by photolysis, or

(d) by reaction with a free radical-producing compound optionally in the presence of an initiator;

(ii) in the case of compound Vb by treatment with a Lewis acid or a base, and

(iii) in the case of compound Vc by treatment with a rhodium- or copper-containing compound;

(D) cyclising a compound of formula VIa, VIb, VIc or VId

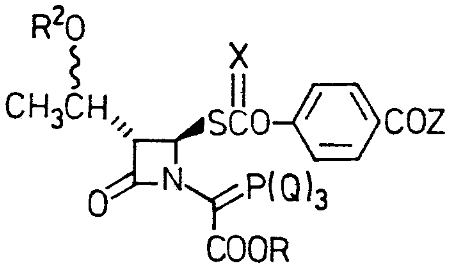

(VIa)

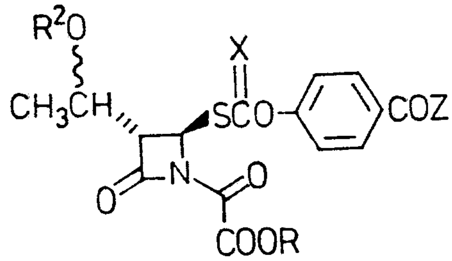

(VIb)

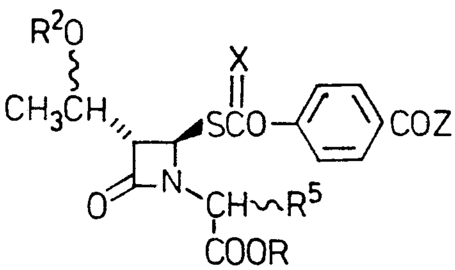

(VIc)

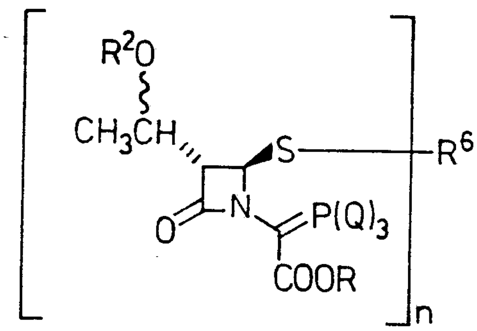

(VId)

in which formulae R, $R^2$ and Z are as defined above,

X represents an oxygen or sulphur atom,

the group $-P(Q)_3$ represents a group derived from a trivalent organophosphorus reagent,

$R^5$ represents a bromine or chlorine atom,

$R^6$ represents Cu(II), Pb(II) or Hg(II), in which case n represents 2, or represents Ag(I), in which case n represents 1,

(i) in the case of compound VIa, by heating compound VIa or allowing compound VIa to stand at room temperature,

(ii) in the case of compound VIb, by treatment with a trivalent oganophosphorus reagent and heating the resulting compound or allowing the resulting compound to stand at room temperature;

(iii) in the case of compound VIc, by treatment with a phosphine and a base, and then heating the resulting compound or allowing the resulting compound to stand at room temperature,

(iv) in the case of compound VId by reaction with a compound of formula VII

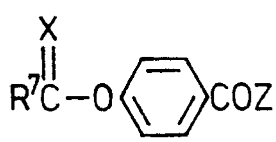

(VII)

in which X and Z are as defined above and

$R^7$ represents an activating group, for example, an activating ester group or a halogen atom, and heating the resulting compound or allowing the resulting compound to stand at room temperature;

(E) cyclising a compound of formula VIIIa or VIIIb

(VIIIa) 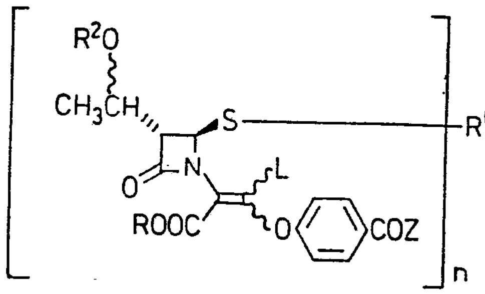 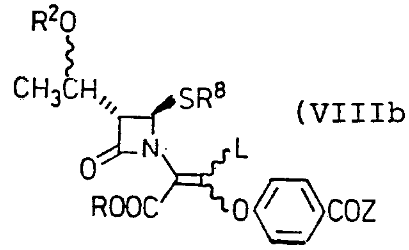 (VIIIb)

in which formulae R, $R^2$, $R^6$, n, and Z are defined as above,

L represents a leaving group, for example, that can be replaced in a nucleophilic displacement reaction, for example, a halogen atom, preferably a bromine or iodine atom; hydroxy group (in which case keto-enol tautomerism can occur); a modified hydroxy group, especially a sulphonate ester group, for example, a sulphonyloxy group of the formula $-OSO_2R_b$ in which $R_b$ represents a straight or branched chain alkyl group having from 1 to 4 carbon atoms, which may be substituted by one or more fluorine atoms, for example, may be fully fluorinated, for example, a $-CF_3$ or $-C_4F_9$ group, or $R_b$ represents a phenyl group that is unsubstituted or is substituted by a p-nitro, p-bromo- or p-methyl group; or L represents a phosphorus-containing group for example

$$-O\overset{\overset{O}{\|}}{P}(R_a)_2 \qquad -S\overset{\overset{O}{\|}}{P}(R_a)_2 \qquad -O\overset{\overset{O}{\|}}{P}(OR_a)_2$$

in which the two groups $R_a$, which may be the same or different, are as defined above, and

$R^8$ represents a hydrogen atom or a sulphur protecting group,

(i) in the case of compound VIIIa, effecting cyclisation, and

(ii) in the case of compound VIIIb, removing the sulphur protecting group, if present, and either reacting the resulting thiol compound with a reagent capable of converting the compound into compound VIIIa, and cyclising compound VIIIa, or cyclising the thiol compound by treatment with a base;

and as desired, and as required carrying out any of one or more of the reactions set out in the further steps (2) to (4) in any appropriate order:

(2) in a compound in which -COZ represents other than a $-CONHR^1$ group carrying out one of the following reactions,

(a) when -COZ is $-CONHR^3$ or $-CONR^3R^3$, removing the protecting group(s) $R^3$;

(b) when -COZ is $COR^4$, reacting the compound with ammonia , a lower alkylamine or a chemical equivalent thereof;

(c) when -COZ is -COOH, activating the -COOH group, and then reacting the resulting compound with ammonia or methylamine;

(d) when -COZ represents -CN, converting -CN to $-CONHR^1$

(3) in a resulting compound of formula Ia, Ib or II in which the free or protected 8-hydroxy group has S-stereochemistry carrying out a stereochemical inversion to give the corresponding 8R compound;

(4) if desired or required, carrying out any one or more of the following steps in any desired order:

a) hydrolysing a 2-carboxylic ester group in a compound to give the corresponding free acid,

b) treating a free acid compound or a salt thereof with an agent capable of forming a 2-carboxylic acid ester, for example, with an alcohol, a phenol or a reactive derivative thereof, to give a 2-carboxylic

acid ester thereof,

c) carrying out an acid- or base-catalysed ester interchange on a 2-carboxylic acid ester to give a different ester of that compound,

d) treating a free acid compound with a base to give a salt at the carboxy group at position 2,

e) treating a free acid compound or 2-carboxylic acid ester having a basic group present with an acid to give an acid addition salt thereof,

f) treating a salt of a compound with an acid to give a free acid of that compound,

g) removing a hydroxy protecting group from a compound having a protected 8-hydroxy group to give the corresponding compound having a free 8-hydroxy group,

h) treating a compound having a free hydroxy group at the 8-position with an organic acid derivative to form an ester at the 8-position.

It will be appreciated that when Z represents an $-NHR^1$, group, then a compound of formula Ia is obtained directly by processes (A) to (E). It is generally preferable to protect a free aminocarbonyl group $-CONHR^1$ during the various reactions involved in the production of a compound of formula Ia. A methylaminocarbonyl group is less reactive, however, and may be carried as such throughout a reaction sequence. Nitrogen protecting groups, their introduction and their removal are well known and are described, for example, by McOmie, Protecting Groups in Organic Chemistry, Plenum Press, London and New York, 1981 and T.W. Greene, Protective Groups in Organic Synthesis, Wiley, New York, 1981.

References herein to "a compound of formula Ib" accordingly include compounds of formula Ia.

The term "pro-drug" group' is used to denote a group that can be removed under physiological conditions.

The term "lower alkyl" is used herein to denote an alkyl group having from 1 to 4 carbon atoms. A lower alkyl group is especially a methyl group.

As indicated in (A) above, a compound of formula Ib may be isolated from compound II which may be in the form of an isomeric mixture comprising compound Ib and the corresponding 5S-isomer. Chromatographic or other techniques known for isolating isomers may be used.

A compound of formula II, which may be obtained by process (B) or by any other method, is preferably converted into a compound of formula Ib by heating, that is to say, a 5R,5S-mixture (compound II) can be converted to predominantly the desired 5R-isomer (compound Ib), or a substantially pure 5S-isomer can be converted into substantially pure 5R-isomer. In the thermal interconversion, 5R⇄5S the position of the solution equilibrium lies more towards formation of the desired 5R-isomer.

The stereochemical inversion is carried out simply by heating compound II, for example, to a temperature within the range of from 30 to 200°C, preferably from 60 to 150°C.

This reaction is generally carried out in a solvent or diluent, or a mixture of two more solvents and/or diluents. It is not generally necessary to ensure that compound II is completely dissolved at the reaction temperature.

The solvent or diluent should be inert under the reaction conditions and may be an ether, for example, diethyl ether, dioxane, tetrahydrofuran, diisopropyl ether or di-n-butyl ether; an aromatic hydrocarbon, for example, toluene or xylene; an ester, for example, ethyl acetate; an aprotic solvent, for example, dimethylformamide or dimethylacetamide, a halogenated hydrocarbon, for example, carbon tetrachloride or chloroform; a ketone, for example, acetone or methyl ethyl ketone; acetonitrile; or a mixture of two or more of such substances. The solvent is chosen with regard

to the nature of the groups -COZ and $R^2O$- and the solubility and stability characteristics conferred by them.

As indicated above, the hydroxy group at position 8 in compound II may be free or protected. It is generally preferable to use a free hydroxy compound II and to introduce any desired ester group, for example, a "pro-drug" group after formation of compound Ib or, preferably, Ia.

The substituent at the 4-position of the phenoxy group in compound II may be a cyano group, a carboxylic acid group, an activated carboxylic acid group or a free or protected aminocarbonyl group. In the case of a protected aminocarbonyl group, conversion to the free aminocarbonyl group or a methylaminocarbonyl group may be carried out before or after the stereochemistry inversion at position 5. If a compound of formula I having a free a methylaminocarbonyl group $-CONHR^1$ is desired, it is preferable to use a compound of formula II having the corresponding aminocarbonyl group as starting material. Conversions of groups at the 4-position are disclosed in more detail below.

The stereochemistry at position 8 in compound II may be R or S, but is preferably R, as this configuration is maintained during the inversion at position 5, resulting in a compound of formula I having the desired 5R,6S,8R stereochemistry without the need for a further inversion.

Accordingly, a particularly preferred compound of formula II has the following structure IIa

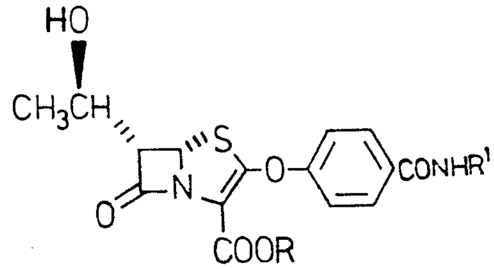

(IIa)

in which R represents a carboxy protecting group, preferably a p-nitrobenzyl group, and $R^1$ is defined as above. For compound IIa, a preferred solvent is acetonitrile. The use of a compound IIa as starting material gives a compound of formula Ic

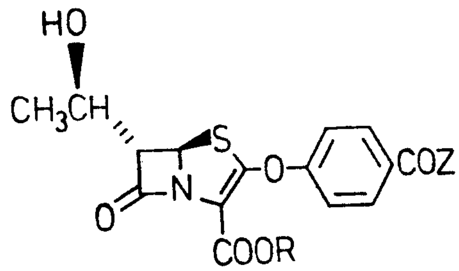

(Ic)

A general advantage of the process described above for the production of compound Ib from a compound II over the processes described previously is that the stereochemistry at position 5 in compound II is not important. As indicated above, the equilibrium 5R$\rightleftharpoons$5S is in favour of the 5R-isomer. Accordingly, it is not necessary to separate the 5-isomers, for example, chromatographically at preceding stages of the reaction sequence. This is a considerable advantage.

Moreover, depending on the nature of the groups R, $R^1$, $R^2$ and Z, we have found that, to varying degrees, the resulting compound Ib is relatively insoluble in liquids that are, to varying degrees, solvents for compound II. This effect is particularly marked when a compound IIa in which $R^2$ is hydrogen is used as the starting material, as the resulting compound Ic is substantially insoluble in solvents in

which the compound IIa is more soluble. Accordingly, the equilibrium between compounds Ic and IIa is driven even more towards compound Ic. Moreover, the relative insolubility of compound Ic means that it can be removed from the reaction mixture very easily, for example, merely by filtration, in very good yield and in substantially pure form, thus also obviating the need for further purification, for example, chromatography.

As indicated above, a compound of formula II may result from process B described briefly above and in more detail below. In some cases depending, for example, on the starting material, reagents and reaction conditions used, the product of process B may already be predominantly the 5R-isomer i.e. a compound of formula Ib. In other cases, the product may be a mixture of 5R,5S-isomers in which the 5S-isomer may predominate. In the case of a mixture, the desired 5R-isomer may be separated from any 5S-isomer, for example, chromatographically, but this is not necessary because of the preferred final thermal inversion.

A compound of formula II may also be produced as described in Reaction Scheme I below.

REACTION SCHEME 1

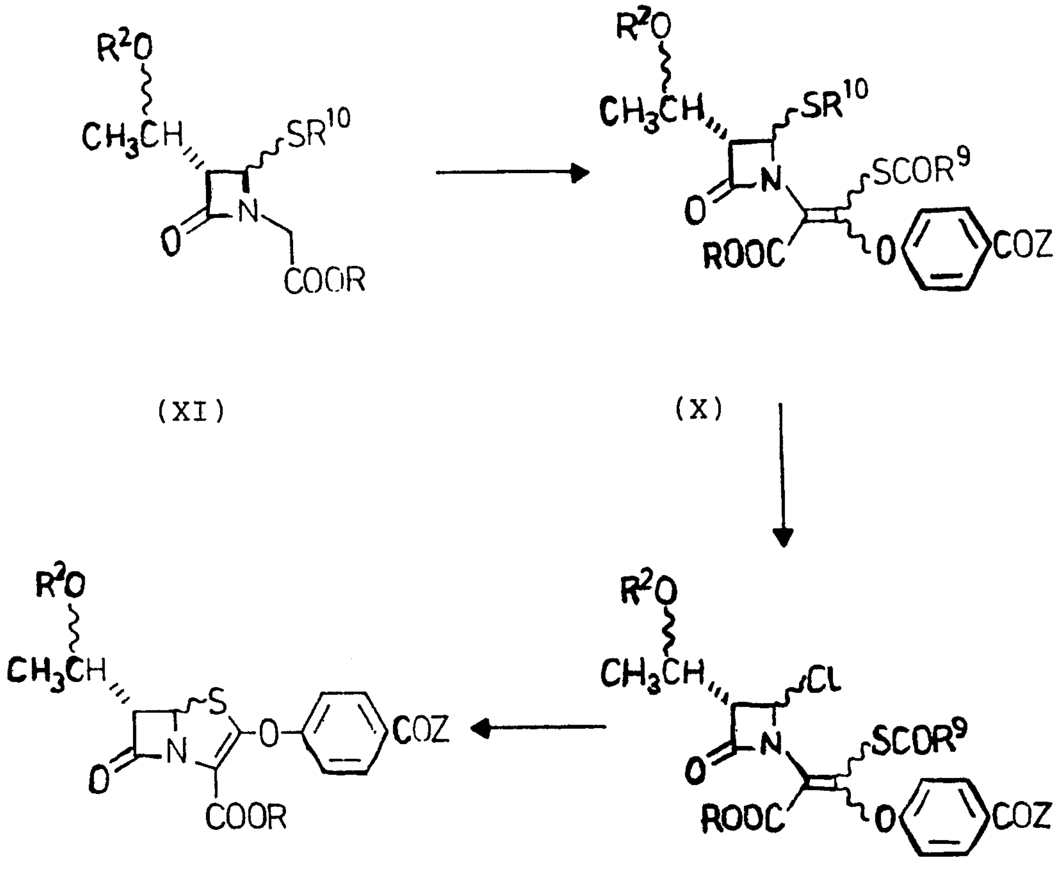

(XI)

(X)

(IX)

(II)

In Reaction Scheme I, Z, R, and $R^2$ are as defined above, and $R^9$ and $R^{10}$ each independently represents a phenyl group or an alkyl group having from 1 to 4 carbon atoms. It is preferable that $R^2$ in compounds IX, X and XI represents a hydroxy protecting group to prevent the hydroxy group from reacting with the various reagents used, and the carboxy group is preferably protected in compounds XI, X, IX and II to prevent it from taking part in any undesired reactions. (In compound II, as indicated above, $R^2$ preferably represents a hydrogen atom, if this is the desired $R^2$ in the final product.)

Certain compounds of formula XI have been described previously see, for example, GB 2 102 798A, GB 2 087 880A, and T. Tanaka et al, J.C.S. Chem. Comm. 1982, (13), 713. Other compounds of formula XI may be prepared analogously.

Compound XI is reacted, in the presence of a base, with a compound of formula XII

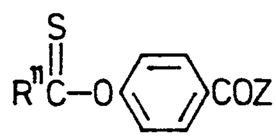

(XII)

in which Z is as defined above, and $R^{11}$ represents a halogen atom, preferably a chlorine atom or an imidazolide group, followed by reaction with an activated carboxylic acid derivative which comprises the group $R^9$ as defined above, for example, with an acyl halide of formula XIII

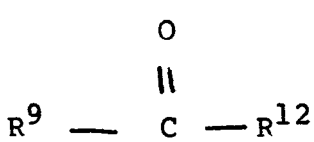

(XIII)

in which $R^9$ is as defined above and $R^{12}$ represents a chlorine or bromine atom.

Many phenyl chlorothionoformates are known, see, for example, Rivier & Schalch, Helv. Chem. Acta., Vol 6, 1923, p605, and Reich & Martin, Chem. Berichte, Vol 98, 1965, p2063. Other compounds of formula XII may be prepared by methods analogous to those described for the preparation of known compounds.

The reaction between compound XI and compound XII is carried out in the presence of a base, preferably having a $pK_a \geqslant 20$, preferably a metallated amine, and examples of preferred bases are lithium diisopropylamide, lithium hexamethyldisilazide, lithium 2,2,6,6-tetramethylpiperidide, lithium cyclohexylisopropylamide, and sodamide.

The reaction is generally carried out in an aprotic solvent, for example, an oxygenated hydrocarbon preferably an ether, for example, diethyl ether, tetrahydrofuran, dioxane, glyme or diglyme. The reaction temperature is, for example, from -120 to +30°C, preferably from -78 to -20°C.

The amount of base used is, for example, from 1 to 3 moles, calculated per mole of compound XI, preferably from 1.5 to 2.5 moles of base. The compound of formula XII is preferably used in an amount of from 1 to 1.5 moles per mole of compound XI, preferably form 1 to 1.1 moles of compound XII per mole of compound XI.

The reaction may be carried out as follows: The base may be added to a stirred solution of compounds XI and XII. Alternatively, to a stirred solution of compound XI under an inert atmosphere is added the base and subsequently a solution of compound XII in the same or a different solvent.

The activated acid derivative, preferably of

formula XIII, is preferably added to the mixture resulting from the reaction of compounds XI and XII, especially in an amount of from 1 to 2 moles calculated on compound XI. The reaction is preferably carried out at a temperature of from -70 to +40°C, adding the compound of formula XIII to the reaction mixture at the temperature at which the reaction between compounds XI and XII took place, and then warming, or working-up at this temperature.

The $-SCOR^9$ group in the resulting compound of formula X may be E or Z to the -COOR group. (The terms E and Z are as defined on page 142 of Allinger et al, "Organic Chemistry" 1971, Worth, New York.) The isomers may be separated for the subsequent reaction, but this is not generally necessary, and the isomeric mixture is generally used as both isomers give a compound of formula II.

The resulting compound X is then chlorinated using an agent capable of splitting a carbon-sulphur bond and of introducing a chlorine atom. Such agents are well known in the art and include, for example, molecular chlorine, sulphuryl chloride, t-butyl hypochlorite and cyanogen chloride.

The reaction is generally carried out at a temperature within the range of from -60 to +20°C. The reaction is generally carried out in a solvent or diluent that is non-protic, and is inert under the reaction conditions, for example, an ether, a hydrocarbon or a halogenated hydrocarbon, for example, dioxane, benzene, chloroform or methylene chloride. A mixture of two or more solvents may be used. Examples of halogenating systems are: chlorine in chloroform, chlorine in benzene and t-butyl hypochlorite in benzene. In the latter two cases, the temperature is preferably form 5 to 20°C, and normally from 5 to

10°C. Generally, 1 to 2 moles of chlorine are used per mole of compound X, cf. S. Kukolja, J. Amer. Chem. Soc. (1971), 93, 6267 and P.C. Cherry, C.E. Newall and N.S. Watson, J.C.S. Chem. Comm. 1979, p.663.

The resulting compound of formula IX may be converted into a compound of formula II in the presence of a base. The base may be inorganic or organic, and may be chosen, for example, from ammonia, or an alkali metal, especially a sodium or potassium, carbonate, bicarbonate, alkoxide or hydroxide; a primary amine, for example, having a $pK_a$ within the range of from 5 to 9, for example, methylamine, ethylamine, aniline or benzylamine; or imidazole, pyridine or a substituted pyridine, for example, an alkyl, amino or alkylamino-substituted pyridine or 4-dimethylaminopyridine. Imidazole, pyridine, ammonia and methylamine are particularly preferred. It will be appreciated that if Z in compound IX represents an activated carboxylic acid group and the base used is ammonia or methylamine, then the activated group can be converted simultaneously into a group $-CONHR^1$.

The reaction is generally carried out in a solvent or diluent, the choice of which is wide, provided that it is inert under the reaction conditions. Examples of solvents and diluents are oxygenated hydrocarbons, for example, alcohols, for example, having up to 4 carbon atoms, for example, ethanol; ethers, for example, having up to 8 carbon atoms, for example, diethyl ether, also tetrahydrofuran and dioxane; ketones, for example, having up to 4 carbon atoms, for example, acetone and methyl ethyl ketone; esters, for example, methyl acetate and ethyl acetate; amd amides, for example, dimethylformamide and dimethylacetamide; also chlorinated hydrocarbons, for example, chloroform,

methylene chloride and carbon tetrachloride; aromatic hydrocarbons, for example, benzene and toluene; and other solvents for example, acetonitrile and nitromethane. A mixture of two or more solvents may be used, and solvents are preferably used in admixture with water, preferably a water-miscible solvent is used in admixture with 5 to 20 % (v/v) water.

The reaction is generally carried out at a temperature within the range of from -20 to 40°C, preferably from 0 to 20°C.

A resulting compound II is then converted into a compound Ib, preferably by heating as described above to produce the desired 5R stereoisomer of formula Ib. If present a hydroxy protecting group may be removed, if desired, or converted into a "pro-drug" group.

A group -COZ other than a group $-CONHR^1$ is converted into the desired group $-CONHR^1$ at an appropriate point. This and other interconversions may be carried out as described briefly above and in more detail below.

As indicated above, the present invention provides a process for the production of a compound of formula Ia

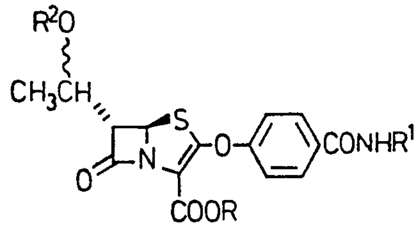

(Ia)

in which R, $R^1$ and $R^2$ are as defined above which comprises producing a compound of formula Ib

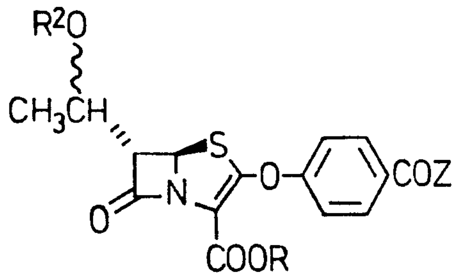

(Ib)

in which R, $R^2$ and Z are defined as above by reacting a compound of formula IV

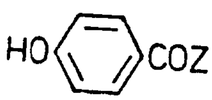

(IV)

in which Z is defined as above with a compound of formula (III)

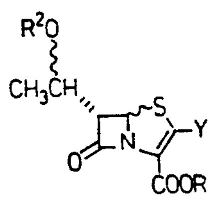

(III)

in which R, $R^2$ and Y are as defined above and, if the resulting compound has the formula II

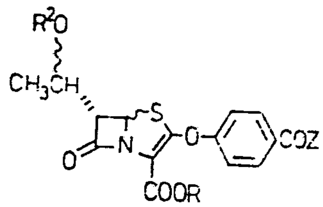

(II)

in which R, $R^2$ and Z are as defined above, converting compound II to compound Ib and/or isolating compound Ib from compound II; and as appropriate and as desired, carrying out any one or more of the reactions described in steps (2) to (4) above, in any appropriate order.

The reaction between compounds III and IV is preferably carried out in the presence of a base, for example, a non-nucleophilic base, for example, a tertiary amine, for example, triethylamine, pyridine or lutidine or an alkali metal hydroxide or alkoxide having from 1 to 4 carbon atoms. A solvent is generally used, for example, a polar solvent, for example, acetonitrile, dimethylformamide, dimethylsulphoxide or dimethylacetamide. The reaction temperature is, for example, within the range of from 0 to 100°C, preferably from 20 to 80°C. A molar excess of the phenol of formula IV may be used, for example, the molar ratio compound III:compound IV is 1:5 to 1:2.

If compound III is a mixture of 5R and 5S isomers the resulting mixture of isomers of formula II may be separated chromatographically or, preferably, may be subjected to a thermal stereochemical inversion as described above. If a substantially pure 5R-isomer of formula III is used, the resulting compound Ib is the corresponding 5R-isomer, also in substantially pure form.

If Z in the resulting compound is other than a free amino or lower alkylamino group, then the group Z is converted into such a

aminocarbonyl group, for example, as described below. This reaction may be carried out at any appropriate point in the series of reactions involved in the production of compound Ia. A protecting group R or $R^2$ present may be removed, or converted into a different, for example, "pro-drug", group. These and other interconversions may be carried out as outlined above and described in more detail below.

Many compounds of formula III are known and may be produced by reacting a compound of formula XIVa or XIVb

(XIVa) 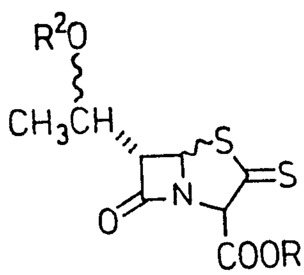

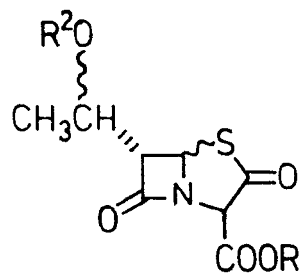 (XIVb)

in which R and $R^2$ are defined as above or a tautomer thereof having an endocyclic double bond with the appropriate reagent to produce the desired compound of formula III. Such reactions are either known <u>per se</u> or may be carried out analogously to known reactions for the production of similar compounds. (The term "known" is used herein to mean in actual use in the art or described in the literature of the art.)

A compound of formula XIVa may be produced by a process as described in our Specification No. 2 087 880A or Specification No. 2 118 546A, and a compound of formula XIVb may be produced as described in DE 2 950 898 or J 56 115 788.

Examples of methods for producing various compounds of formula III from a compound of formula XIVa, from a compound of formula XIVb, or from another starting material are as

follows:

(i) Where Y represents certain phenoxy groups, a compound of formula III may be produced as described in our UK Specification No. 2 102 798A, and where Y represents certain phenylthio groups, as described in EP 0 002 210A. Other compounds of formula III may be prepared analogously.

(ii) Where Y represents a group

$$-X\overset{O}{\overset{\|}{P}}(ORa)_2 \qquad \text{or} \qquad -X\overset{O}{\overset{\|}{P}}(Ra)_2$$

in which X and $R_a$ are defined as above, a compound of formula III may be produced by reacting compound XIVa or compound XIVb with a phosphorus halide of formula XVIa or XVIb

$$\text{(XVIa)} \quad R^{13}—\overset{O}{\overset{\|}{P}}(ORa)_2 \qquad\qquad R^{13}—\overset{O}{\overset{\|}{P}}(Ra)_2 \quad \text{(XVIb)}$$

in which $R^{13}$ represents a halogen atom and Ra is as defined above.

The reaction is preferably carried out in the presence of a base, for example, a base having a pK>7, for example, a tertiary amine base, for example, triethylamine, diisopropylethylamine, or pyridine. The reaction is preferably carried out in a solvent or diluent that is inert under the reaction conditions, for example, a halogenated aliphatic or aromatic hydrocarbon, for example, chloroform or dichloromethane; a hydrocarbon, for example, toluene or xylene; an ether, for example, diethyl ether, tetrahydrofuran or dioxane; or acetonitrile. The reaction temperature is, for example, within the range of form -40 to +50°C. The resulting phosphorylated

compound of formula III is not generally isolated but is preferably reacted _in situ_ with the compound of formula IV.

(iii) Where Y represents a group $-OSO_2R_b$ in which $R_b$ is defined above, a compound of formula III may be produced by reacting a compound of formula XIVb with a compound of formula

$$Cl-SO_2R_b$$

in which $R_b$ is as defined as above, this reaction generally being carried out in the presence of a base, for example, a base as described in section (ii) immediately above, and at a temperature generally within the range of from $-78^o$ to $+50^oC$, preferably from $-30^o$ to $0^oC$, the product generally not being isolated but reacted subsequently _in situ_.

(iv) Where Y represents a group

$$\overset{O}{\uparrow}{-SR_a}$$ or $-SO_2R_a$ in which $R_a$ is as defined above, by oxidising the corresponding group $-SR_a$

The oxidation may be carried out by means of a chemical oxidising agent, for example, a peracid, for example, peracetic acid or _m_-chloroperbenzoic acid; hydrogen peroxide; an alkylhydroperoxide, for example, _t_-butyl hydroperoxide; a permanganate, for example, potassium permanganate; a perborate, for example, sodium perborate; or a persulphate, for example, potassium persulphate.

The oxidation is carried out in a solvent or diluent that is inert under the reaction conditions, for example, a chlorinated hydrocarbon, for example, dichloromethane or chloroform or an oxygenated hydrocarbon, for example, diethyl ether, tetrahydrofuran or ethyl acetate. Particularly preferred solvents

are dichloromethane and ethyl acetate.

The oxidation is generally carried out at a temperature within the range of from -70 to +20°C, preferably from -40 to 0°C.

The starting penem material having an $-SR_a$ group may be produced by as described in our UK Specification No. 2 102 798A or by a process analogous thereto.

(v) Where Y represents the group $-O\overset{O}{\overset{\|}{C}}CF_3$, a compound of formula III may be produced by reacting compound XIVb with trifluoroacetic anhydride in the presence of a base.

An advantage of the process described above for the production of a compound of formula Ia is that compounds of formula III, in which the group Y does not represent a $-CONHR^1$ group, may be produced under conditions in which the desired group $-CONHR^1$ is unstable. The group can be converted into the desired $-CONHR^1$ group subsequently. This leads to higher yield and ease of handling the intermediate compounds.

As indicated above, the present invention provides a process for the production of a compound of formula Ia

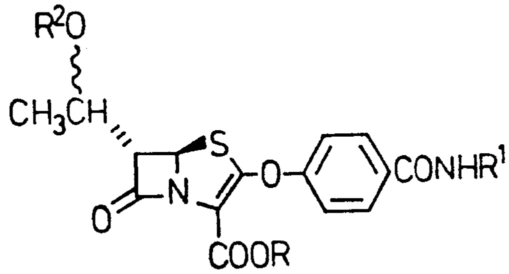

(Ia)

in which R, $R^1$ and $R^2$ are defined as above, which comprises producing a compound of formula Ib

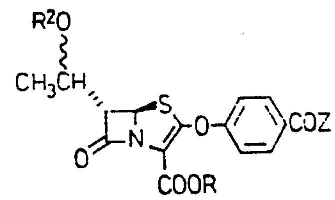

(Ib)

in which R, $R^2$ and Z are defined as above by cyclising a compound of formula Va, Vb or Vc

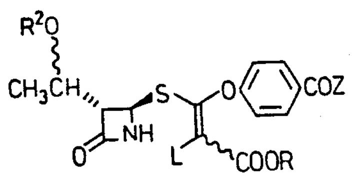

(Va)

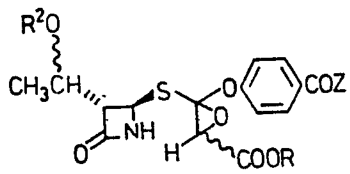

(Vb)

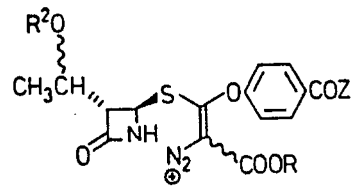

(Vc)

in which R, $R^2$ and Z are as defined above, and $L^1$ represents a chlorine or bromine atom;

(i) in the case of compound Va eliminating $HL^1$

(a) by treatment with a base a metal or a metal-containing reagent, a base and a metal or a base and a metal-containing reagent,

(b) by electrolysis,

(c) by photolysis, or

(d) by reaction with a free radical-producing

compound optionally in the presence of an initiator;

(ii) in the case of compound Vb by treatment with a Lewis acid or a base, and

(iii) in the case of compound Vc by treatment with a rhodium- or copper-containing compound and, if desired or required,

(B) carrying out step (2) as defined above and, as desired and as required, carrying out any one or more of the reactions defined in steps (2) to (4) above, in any appropriate order.

In process (i) (a) or (ii) the base used to cyclise compound Va or Vb preferably has a pK$\geqslant$9 and is, for example, a proton sponge, a tertiary amine, for example, triethylamine, an alkali metal alkoxide, for example, sodium or potassium methoxide or sodium or potassium ethoxide, or lithium hexamethyldisilazide or lithium diisopropylamide.

A metal used to bring about cyclisation of compound Va is, for example, copper, palladium or nickel and a metal-containing reagent is, for example, CuCl, CuBr, CuI, $NiCl_2$, $PdCl_2$ or a metal-containing complex, for example, cuprous iodide-dimethyl sulphide complex, cuprous bromide-dimethyl sulphide complex, cuprous iodide-tri-n-butylphosphine complex, tetrakis(triphenylphosphine) Pd(0), tetrakis(triphenylphosphine) Ni(0), bis(cyclooctadienyl)nickel and bis[1,2-di-(diphenylphosphino)ethane]nickel dichloride. Palladium (0) metal is particularly prefered.

The reaction is preferably carried out in a solvent or diluent, inert, non hydroxylic solvents being prefered. Examples of such solvents are tetrahydrofuran, hexamethylphosphoramide, and dimethoxyethane. The reaction temperature is, for example, in the range

of from -78°C to room temperature.

When compound Va is caused to cyclise by electrolysis, the electrodes are, for example, graphite, or a metal, for example, copper or platinum, in wire, plate or gauze form. The electrolysis cell used is generally divided by a semi-permeable membrane, for example, by a frit covered with a gel comprising 0.1 M lithium perchlorate in dimethyl formamide and methylcellulose, or by a perfluorinated exchange membrane.

The supporting electrolyte may be selected from those in conventional use in electrolysis of organic compounds, for example, lithium perchlorate, tetraethylammonium perchlorate, tetraethylammonium *p*-toluenesulphonate or tetrabutylammonium tetrafluoroborate. The solvent may also be one in conventional use, for example, acetonitrile or aqueous acetonitrile, or an alcohol, for example, methanol. The temperature may be, for example, within the range of from -10 to +30°C, for example, from 0 to 20°C.

A photolytically induced cyclisation of a compound of formula Va is preferably carried out using a medium or high pressure mercury lamp, and in a solvent, for example, an aliphatic hydrocarbon, for example, pentane or hexane, an ether, for example, diethyl ether, or an alcohol, for example, ethanol.

A free radical-producing reagent may be used to effect cyclisation of compound Va, for example, an alkyltinhydride, for example, tri-n-butyltin hydride preferably in the presence of an initiator, for example, azabisisobutyronitrile or a peroxide. The reaction is preferably carried out in the presence of a solvent, for example, an aliphatic or aromatic hydrocarbon, for example, hexane, xylene or toluene, a halogenated hydrocarbon, for example,

dichloromethane or chloroform, or an ether, for example, diethyl ether, dioxane or tetrahydrofuran. The reaction temperature is generally within the range of from 30 to 100°C, preferably from 60 to 80°C.

According to reaction (ii), the epoxide Vb is cyclised by treatment with either a Lewis acid, for example, trimethylaluminium, trifluoroacetic acid, borontrifluoride-etherate, aluminium trichloride, tin (IV) chloride or p-toluenesulphonic acid, or with a base generally having a pK$\geqslant$9, for example, as described above. The reaction is generally carried out in a solvent, for example, a hydrocarbon, for example, benzene or toluene, an ether, for example, diethyl ether, tetrahydrofuran or dioxane, a halogenated hydrocarbon, for example, dichloromethane or chloroform, an aprotic solvent, for example, dimethylformamide, or dimethylacetamide, or acetonitrile.

According to reaction (iii), the diazo compound Vc is cyclised in the presence of a copper- or rhodium-containing compound. A rhodium-containing compound is, for example, rhodium acetate, and a copper-containing compound is, for example, copper-bis(acetylacetonate) cf, for example, D.M. Brunwin et al J. Chem. Soc. 1971, 3756. The reaction is generally carried out in a solvent, for example, an aromatic hydrocarbon, for example, toluene, xylene or, especially benzene, a chlorinated hydrocarbon, for example, dichloromethane or carbon tetrachloride, or an ether for example, tetrahydrofuran or dioxane. The reaction temperature is generally within the range of from 30 to 100°C, preferably from 40 to 80°C.

Any one or more of the reactions described in steps (2) to (4) above may be carried out on a resulting compound, if desired or required.

Compounds of formula Va, Vb and Vc may be produced as shown in the Reaction Scheme II below.

REACTION SCHEME II

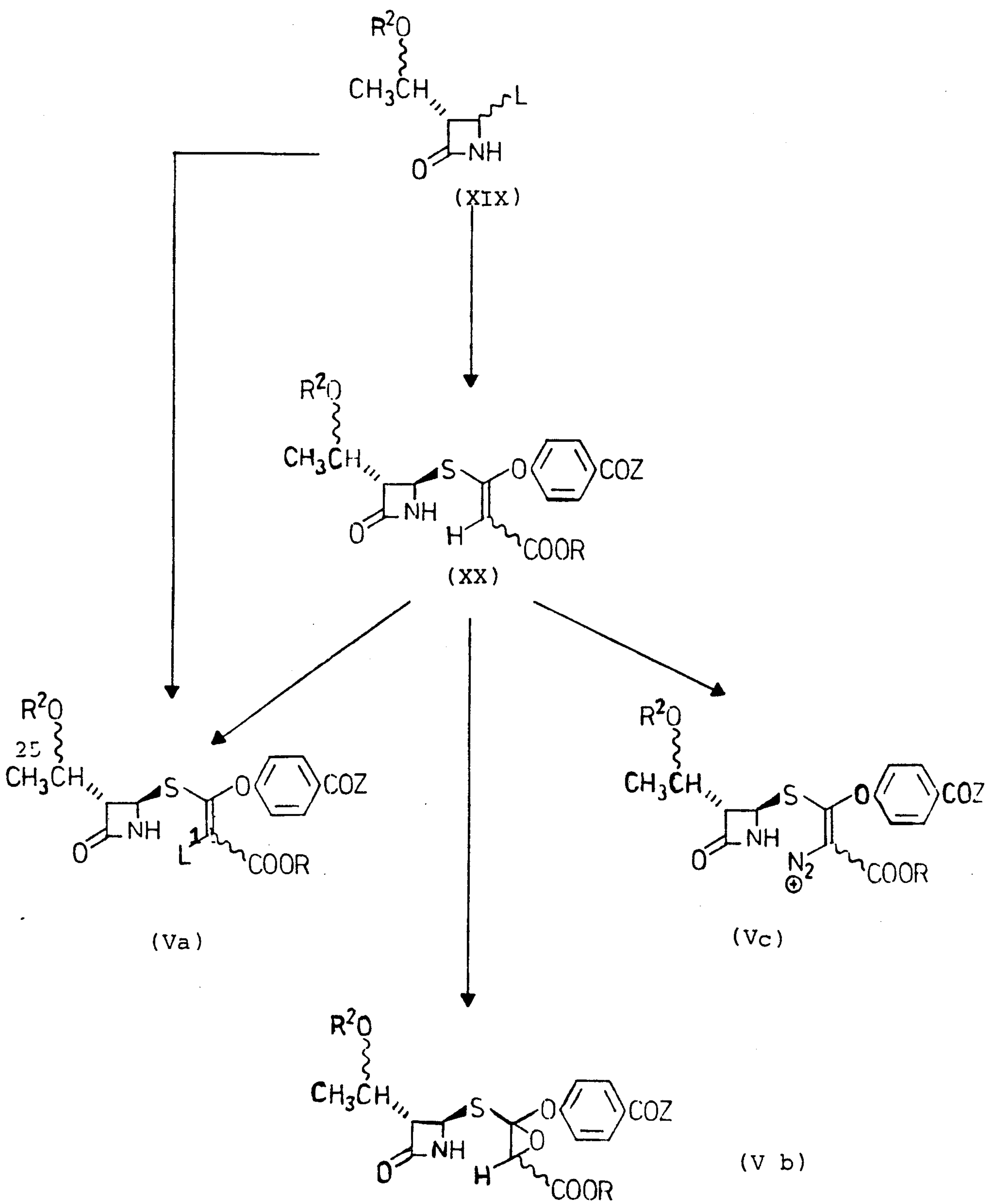

(XIX)

(XX)

(Va)

(Vc)

(V b)

in which R, $R^2$, L and Z are defined as above.

Certain compounds of formula XIX are known, for example, when $R^2$ represents a dimethyl-t-butylsilyl group (Belgian Patent Specification No. 882 764), and when $R^2$ represents a p-nitrobenzyloxycarbonyl group (EP 0 002 210A). Other compounds of formula XIX may be prepared analogously.

When compound XIX is reacted with a compound of formula XXIa

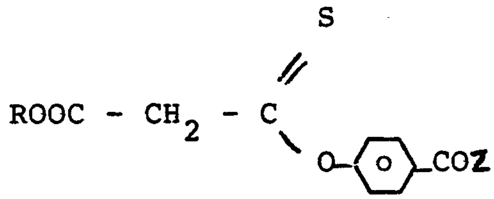

( XXIa )

in which R and Z are defined as above, compound XX is obtained. This reaction is generally carried out in the presence of a base, for example, an alkali metal alkoxide, sodium hydride or a tertiary amine base, for example, sodium methoxide, or triethylamine. The reaction is generally carried out in a solvent or diluent, for example, acetonitrile or hexamethylphosphoramide, an alcohol, for example, methanol, an ether, for example, tetrahydrofuran or a chlorinated hydrocarbon, for example, dichloromethane. A mixture of two or more solvents may be used, for example, a mixture of hexamethylphosphoramide and tetrahydrofuran. The reaction temperature is for example, in the range of from $0^oC$ to room temperature.

Compound XX may then be reacted with the appropriate reagent to give a compound of formula Va, Vb or Vc:

Compound Va may be produced by reacting compound XX with a halogenating agent, for example, bromine, N-chlorosuccinimide, N-bromosuccimide, N-bromoacetamide, or pyridine hydrobromide perbromide.

The reaction may be carried out in the presence of a base, for example, an amine base, for example, triethylamine or pyridine. The reaction is generally carried out in a solvent or diluent, for example, an ether, for example, tetrahydrofuran or diethyl ether, an aromatic hydrocarbon, for example, benzene, or a chlorinated hydrocarbon, for example, dichloromethane, at a reaction temperature, for example, within the range of from -70 to 60°C.

Compound XX may be converted into the epoxide Vb by reaction with a peracid, for example, peracetic, m-chloroperbenzoic, mono-perphthalic or trifluoroperacetic acid, t-butyl hydroperoxide, or hydrogen peroxide optionally in the presence of a base. In the case of t-butyl hydroperoxide a metal agent is also often used, for example, vanadyl acetoacetonate, or a salt of vanadium (V) or of molybdenum (VI). The reaction is generally carried out in a solvent or diluent, for example, an ester, for example, ethyl acetate, a chlorinated hydrocarbon, for example, dichloromethane or chloroform, or an aromatic hydrocarbon, for example, toluene, and at a temperature, for example, within the range of from -70 to +40°C, preferably from -20 to +20°C.

Compound Vc may be produced from compound XX by diazo transfer, for example, using p-dodecylbenzene-sulphonyl azide or p-toluenesulphonyl azide which may be polymer-bound. This reaction is generally carried out in the presence of a base, preferably an organic base, for example, a tertiary amine, for example, triethylamine or diisopropylethylamine or DBU. The reaction is preferably carried out in a solvent that is inert under the reaction conditions used, for example, acetonitrile or a chlorinated hydrocarbon, for example, dichloromethane. The reaction temperature is generally

within the range of from -20 to +20°C.

Compound Va may be obtained directly from compound XIX by treating a compound of formula XXIb

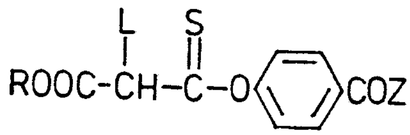

(XXIb)

in which L, R and Z are defined as above, with a base, preferably having a pK≥9, for example, as described above and preferably triethylamine and then incorporating compound XIX. A solvent is generally used, for example, an ether, for example, diethyl ether, dioxane or tetrahydrofuran, a hydrocarbon, for example, benzene or toluene, a chlorinated hydrocarbon, for example, dichloromethane, or a mixture of two or more solvents. The reaction temperature is generally within the range of from -20 to +50°C.

The conversion of a resulting compound Ib into a compound Ia, and any of the further various optional interconversions desired may be carried out at an appropriate point as described briefly above and in more detail below.

One advantage of the processess described above for the production of a compound of formula Ia using a compound of formula Va, Vb or Vc is that a strong base is not used, so the possibility of undesired side reactions is reduced. A further advantage is that the number of steps from the initial compound of formula XIX is small, indeed, compound Ia can be produced from compound XIX via compound Va in only two steps. For the other routes only three steps are involved.

As indicated above, the present invention provides a process for the production of a compound of formula Ia

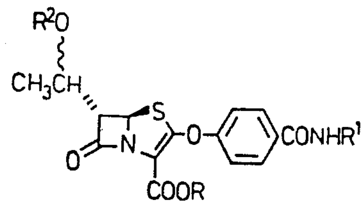

(Ia)

in which R, $R^1$ and $R^2$ are defined as above, which comprises producing a compound of formula Ib

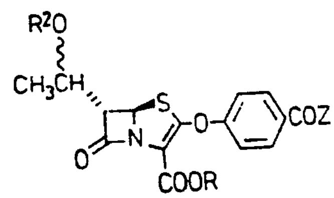

(Ib)

in which R, $R^2$ and Z are defined as above by cyclising a compound of formula VIa, VIb, VIc or VId

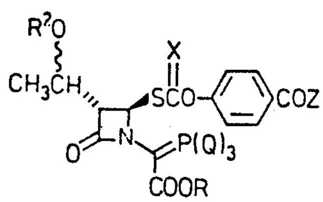

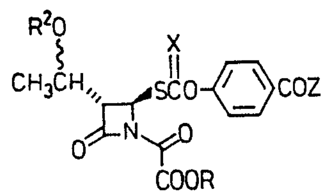

(VIa) (VIb)

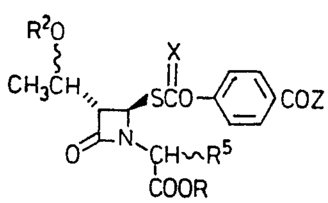

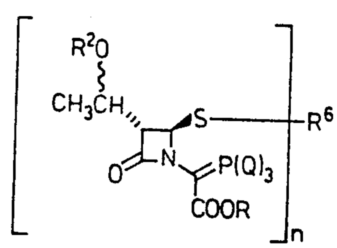

(VIc) (VId)

in which formulae R, $R^2$, $R^5$, $R^6$, X, Z, n and

$P(Q)_3$ are as defined above,

(i) in the case of compound VIa, by heating compound VIa or allowing compound VIa to stand at room temperature,

(ii) in the case of compound VIb, by treatment with a trivalent oganophosphorus reagent and the heating the resulting compound or allowing the resulting compound to stand at room temperature;

(iii) in the case of compound VIc, by treatment with a phosphine followed by treatment with a base, and then heating the resulting compound or allowing the resulting compound to stand at room temperature;

(iv) in the case of compound VId by reaction with a compound of formula VII

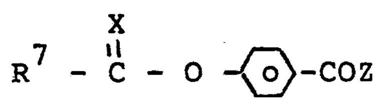

(VII)

in which $R^7$, X and Z are as defined above, and heating the resulting compound or allowing the resulting compound to stand at room temperature; and as required and as desired, carrying out any one or more of the reactions described in steps (2) to (4) above, in any appropriate order.

The procedures described in (i) to (iv) above are each generally carried out in a solvent, for example, an aromatic hydrocarbon, for example, benzene, toluene or xylene, a chlorinated hydrocarbon, for example, dichloromethane, chloroform or 1,2-dichloroethane, an ether, for example, dioxane, tetrahydrofuran or di-*n*-butyl ether. The reaction in each case is generally carried out at a temperature within the range of from

20°C to 140°C, preferably from 80° to 110°C.

In each case the reaction is generally carried out under an inert atmosphere, for example, of nitrogen and, if desired in the presence of hydroquinone, for example, in an amount 0.01 to 0.1%, % mole ratio calculated on compound VIa, VIb or VIc. Each reaction may be carried out under high dilution. In those cases where X in a compound of formula VIb used is sulphur, the organophosphorus compound is preferably added slowly to the reaction mixture comprising the compound of formula VI used.

Further details of the organophorus reagents used are given below.

Compounds of formulae VIa, VIb, VIc and VId may be produced as described in Reaction Scheme III below:

REACTION SCHEME III

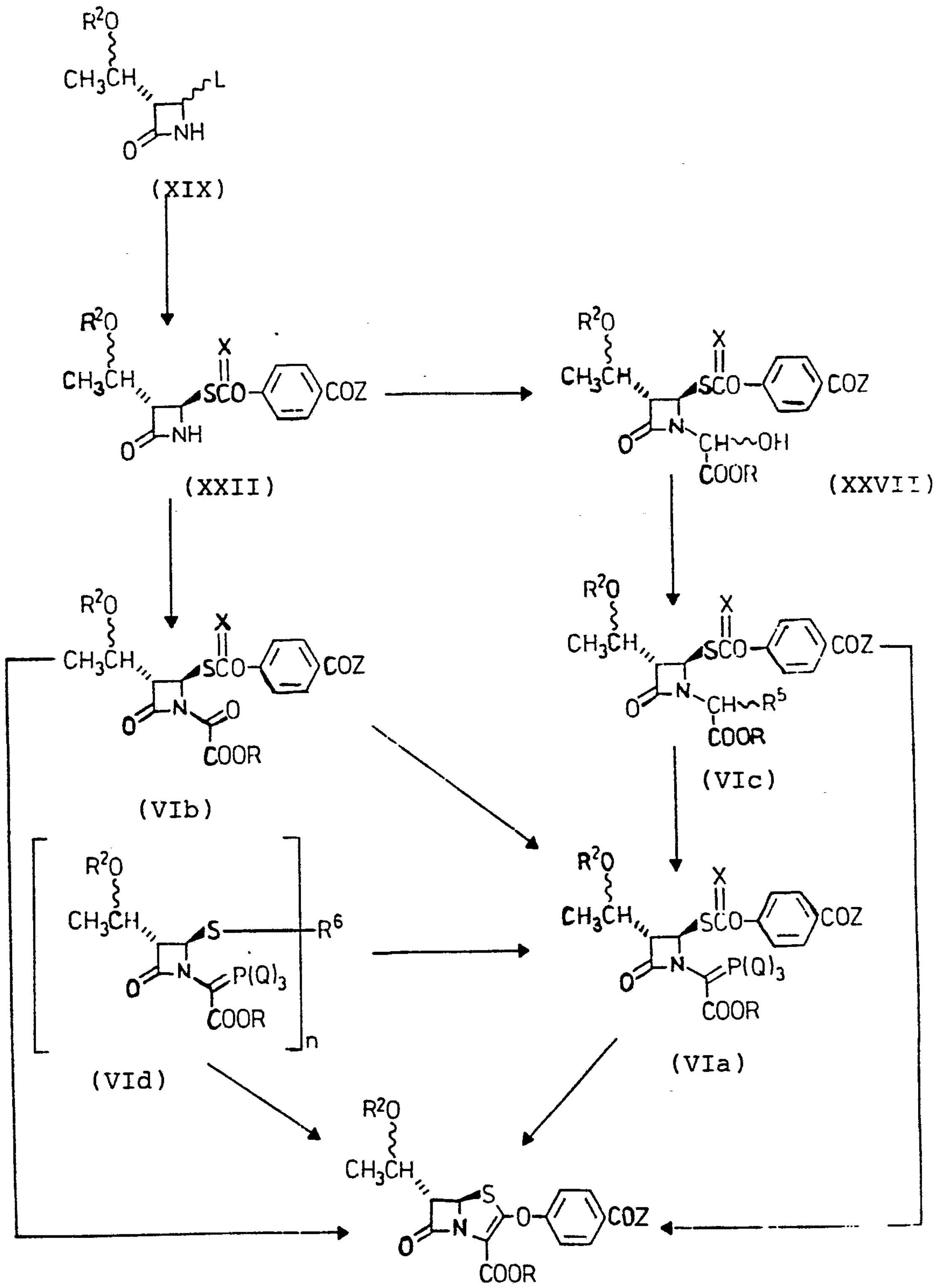

(XIX)
(XXII)
(XXVII)
(VIb)
(VIc)
(VId)
(VIa)

In Reaction Scheme III, R, $R^2$, $R^5$, Z, L and $P(Q)_3$ are defined as above.

Certain compounds of formula XIX are known, for example, when $R^2$ represents a dimethyl-t-butylsilyl group (Belgian Patent Specification No. 882 764), and when $R^2$ represents a p-nitrobenzyloxycarbonyl group (EP 0 002 210A). Other compounds of formula XIX may be prepared analogously.

A compound of formula XXII may be prepared by reacting a compound of formula XIX with a compound of formula XXIII

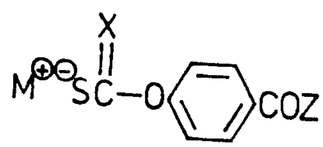

(XXIII)

in which X and Z are defined as above, and M represents an alkali metal or alkaline earth metal atom, or an ammonium group that is unsubstituted or substituted by, for example, one to four groups selected from alkyl groups having from 1 to 4 carbon atoms.

In this process, the reaction is generally carried out in a solvent that is inert under the reaction conditions, for example, in an ether, for example, diethyl ether, dioxane or tetrahydrofuran, an aromatic hydrocarbon, for example, benzene, toluene or xylene, or a halogenated hydrocarbon, for example, chloroform, carbon tetrachloride or dichloromethane.

Compounds of formula XXIII in which M represents an alkali metal or alkaline earth metal atom may be prepared by reacting a compound of formula XXIV

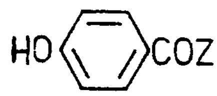

(XXIV)

in which Z is defined as above in the presence of a base comprising the radical M, for example, an alkali metal hydroxide or ammonium hydroxide with carbon disulphide or carbon oxysulphide.

A compound of formula XXII may then be acylated using a compound of formula XXV

$COR^{14}$
|
COOR

(XXV)

in which R is as defined above and $R^{14}$ represents a group that can be displaced by the azetidinone nitrogen in the compound of formula XXII to give a compound of formula VIb.

A group $R^{14}$ is, for example, a halogen atom, an imidazolide group, or a mixed anhydride group, for example, a group -OCORc or -OC(O)ORc in which Rc represents a straight or branched chain alkyl group having from 1 to 4 carbon atoms. $R^{14}$ preferably represents a halogen atom, especially a chlorine atom.

The reaction between compound XXII and the acylating agent of formula XXV is carried out in the presence of a base, for example, a tertiary amine, preferably a trialkylamine (each alkyl moiety having from 1 to 4 carbon atoms and having a straight or branched chain, the three alkyl moieties being the same or different), and especially triethylamine or ethyldiisopropylamine; or pyridine or a substituted pyridine, for example, 4-dimethylaminopyridine.

Particularly preferred bases are triethylamine and ethyldiisopropylamine. A mixture of two or more bases may be used, and a base may be polymer-bound.

In addition to an organic base as described above, it is preferable to include an acid binding agent in the reaction mixture. Examples of acid binding agents are alkali metal and alkaline earth metal carbonates and bicarbonates, and organic epoxides, for example, propylene oxide. Calcium carbonate is a commonly-used binding agent.

In general, for each mole of compound XXII there is used from 1 to 2 moles of the acylating agent XXV, from 1 to 4 moles of an amine base (or a total of from 1 to 4 moles of a mixture of amine bases), and from 0 to 10 moles of the acid binding agent, if present.

The compound of formula XXII and the acylating agent of formula XXV are generally reacted in a solvent or diluent that is inert to the reaction, for example, in a halogenated hydrocarbon, for example, dichloromethane or chloroform, in an oxygenated hydrocarbon, for example, an ether, for example, tetrahydrofuran, dioxane or diethyl ether, or in an aromatic hydrocarbon, for example, benzene or toluene. The reaction is generally carried out at a temperature within the range of from -20 to +60$^{o}$C, preferably from 0 to 20$^{o}$C.

Compound VIb, the product of the acylation reaction, may be isolated if desired, but is generally reacted without further purification with a trivalent organophosphorus reagent to give a compound of formula VIa or to give a compound of formula Ib directly.

Examples of trivalent organophosphorus reagents which may be used to convert compound VIb to compound VIa or compound Ib are phosphites and phosphoramides, for example, of the following formulae XXVIa and XXVIb,

and phosphines of formula XXVIc in combination with phosphites of formula XXVIa

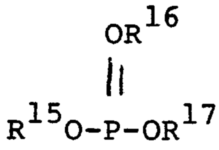
(XXVIa)

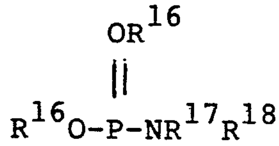
(XXVIb)

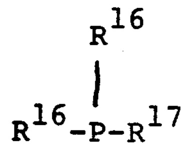
(XXVIc)

in which formula $R^{15}$, $R^{16}$, $R^{17}$, and $R^{18}$, which may be the same or different, each represents a straight or branched chain alkyl group having from 1 to 6 carbon atoms or a phenyl group which may be unsubstituted or substituted, for example, by a methyl group, especially a para-methyl group; also cyclic trialkyl phosphites, each alkyl moiety having from 1 to 4 carbon atoms, for example, of formula XXVId

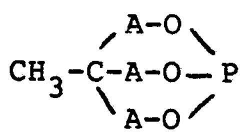
(XXVId)

in which each group A denotes an alkylene group having from 1 to 4 carbon atoms; and also catechol phosphites and catechol dimer phosphites, for example, of the following formulae XXVIe and XXVIf, respectively:

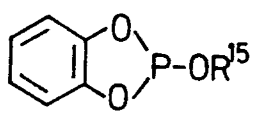
(XXVIe)

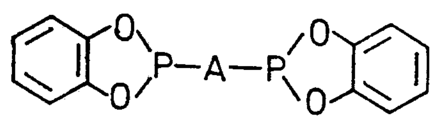
(XXVIf)

in which $R^{15}$ and A are defined as above. A trivalent organophosphorus compound may be bound to an inert polymer.

A preferred trivalent organophosphorus reagent is

a phosphite or phosphoramide of formula XXVIa or XXVIb respectively, or a combination of a phosphine of formula XXVIc with a phosphite of formula XXVIa. Particularly preferred are the trialkyl phosphites, and especially trimethyl phosphite and triethyl phosphite.

When the organophosphorus reagent used is a phosphite or phosphoramide, generally from 2 to 3 moles thereof are used per mole of compound VIb. When a phosphine is chosen, from 1 to 3 moles are generally used per mole of compound VIb, in combination with a phosphite, generally 1 mole thereof. The phosphite is preferably added slowly to a mixture of the phosphine and the compound VIb.

As described above, a resulting compound of formula VIa may be isolated and then cyclised to a compound of formula Ib, or a compound of formula VIb may be reacted with the organophosphorus reagent and cyclised *in situ* to give a compound Ib. In the latter case, the reaction may proceed via compound VIa or via another intermediate.

The cyclisation reaction is generally carried out in a refluxing solvent. When X in compound VIb or compound VIa represents a sulphur atom, preferred solvents are aromatic hydrocarbons, for example, benzene, toluene and xylene, and halogenated hydrocarbons, for example, dichloromethane, chloroform and 1,2-dichloroethane. When X in compound VIa or VIb represents an oxygen atom, preferred solvents are benzene, toluene and xylene. An ether, for example, di-*n*-butyl ether, may be used as solvent when X is a sulphur atom or an oxygen atom.

In a compound of formula VIa, it can be seen that the group $=P(Q)_3$ is derived from the trivalent organophosphorus reagent or mixture of reagents used in its production, which reagent or mixture of reagents is

preferably selected from the reagents of formula XXVIa to XXVIf described above. As mentioned above, the organophosphorus reagent may be polymer-bound, in which case a resulting compound of formula VIa will also be polyer-bound.

Compound Ib may be produced from compound XXII by an alternative route via compounds XXVII, VIc and optionally VIa as shown in Reaction Scheme III.

By this route, a compound of formula XXII is reacted with a glyoxylic ester of formula XXVIII

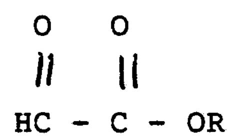 (XXVIII)

in which R is as defined above, or with a reactive derivative thereof, for example, a hydrate or hemiacetal, a hemiacetal preferably being formed with an alcohol having from 1 to 4 carbon atoms, for example, ethanol.

When a hydrate of a compound of formula XXVIII is used, the water formed during the reaction is preferably removed, for example, azeotropically or by use of a dehydrating agent, for example, a molecular sieve.

The reaction is generally carried out in a solvent or diluent that is inert under the reaction conditions, for example, an ether, for example, tetrahydrofuran, dioxane or diethyl ether, an aprotic solvent, for example, dimethylacetamide or dimethylformamide, or an aromatic hydrocarbon, for example, benzene or toluene. A mixture of two or more solvents may be used. The reaction is generally carried out at a temperature within the range of from 0 to 100°C. Preferably from 1 to 2 moles of the glyoxylate derivative are used per

mole of compound XXII.

The resulting compound of formula XXVII is obtained as a mixture of the R- and S-isomers at the >CH-OH group. These isomers may be separated, if desired, but the R,S-mixture is generally used for the next reaction.

In the next step, the alcohol of formula XXVII is converted into a halide of formula VIc. The halogenation may be carried out in a conventional manner using an appropriate agent, for example, thionyl chloride or bromide, phosphorus oxychloride or oxybromide, or a phosphorus halide, for example, phosphorus pentachloride or pentabromide, or a mixture of two or more thereof.

The reaction is preferably carried out in the presence of a base, for example, a heterocyclic base, for example, pyridine, 4-dimethylaminopyridine, or lutidine, or a trialkylamine, for example, triethylamine or diisopropylethylamine. The base may be polymer-bound.

The reaction is generally carried out in a solvent, for example, an ether, for example, diethyl ether or dioxane. The reaction temperature is generally within the range of from -40 to +40°C, preferably room temperature and, if desired, the reaction may be carried out under an inert atmosphere.

The resulting compound of formula VIc is obtained as a mixture of the R- and S-isomers at the >CH-Cl or >CH-Br group. As in the case of compound XXVII, the isomeric mixture may be separated into the individual isomers, but generally the R,S-mixture is used.

A compound of formula VIc may be converted into a compound of formula Ib. This reaction may proceed via a compound of formula VIa or via another intermediate. If desired, a compound of formula VIc may be treated with

a phosphine and a mild base to give a compound of formula VIa, which may then be isolated and cyclised to give a compound of formula Ib.

A compound of formula VIc may be converted into a compound of formula Ib or of formula VIa by reaction at an appropriate temperature as described above with a phosphine of formula XXVIc, for example, triphenylphosphine.

The reaction is preferably carried out in the presence of a base, for example, an alkali metal carbonate or bicarbonate, or an organic amine, for example, a tertiary amine, for example, pyridine or a pyridine derivative, or a trialkylamine, for example, triethylamine or diisopropylethylamine. The reaction is generally carried out in a solvent that is inert under the reaction conditions, for example, an aromatic hydrocarbon, for example, benzene or toluene, or an ether, for example, diethyl ether, tetrahydrofuran or dioxane.

The reaction temperature is generally within the range of form -10 to 150°C. As mentioned above, the use of lower temperatures, for example, room temperature or below, generally enables a compound VIa to be isolated, whereas higher temperatures, for example, above room temperature, generally lead to compound Ib directly.

A compound of formula Ib may be produced by a third method, which is also shown in Reaction Scheme III. This method comprises reacting a heavy metal mercaptide of formula VId

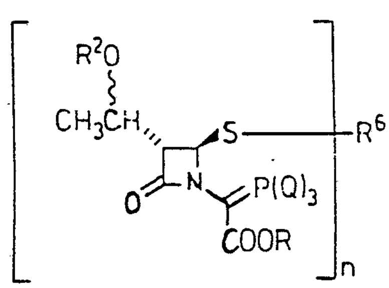

(VId)

in which $=P(Q)_3$, $R^2$ and $R^6$ and are defined as above, with a compound of formula XIIa

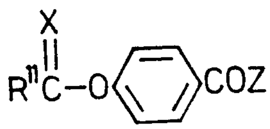

(XIIa)

in which $R^{11}$, Z and X are defined as above.

A compound analogous to formula VId but in which Q represents a phenyl group is described in GB 2 042 515A, which also describes a process for the production thereof. The present compound of formula VId may be produced analogously, using the appropriate trivalent organophosphorus reagent.

Compounds VId and XIIa are generally reacted in a solvent, for example, an ether, for example, diethyl ether or dioxane, an aromatic hydrocarbon, for example, benzene, toluene or xylene, an ester, for example, ethyl acetate, a halogenated hydrocarbon, for example, dichloromethane or chloroform, or an aprotic solvent, for example, dimethylformamide or dimethylacetamide. The reaction temperature is generally within the range of from -40 to 100°C, preferably from 0 to 40°C.

Compound VId may be converted to compound Ib either via compound VIa or via another intermediate, compound VIa optionally being isolated and then converted to compound Ib. Lower temperatures, for example, from -40°C to room temperature, generally enable compound VIa to be isolated, whereas higher temperatures, for example, from room temperature to 150°C, generally lead to compound Ib.

In all the various intermediates involved in the production of a compound of formula Ib by the procedures described in Reaction Scheme III the 8-hydroxy group and the 2-carboxy group, when present, are preferably protected, with removal of the protecting groups preferably being delayed until after compound Ib has been formed and, if appropriate, until any conversions of groups R and/or $R^2$, have been carried out.

Compound VIb may be produced by a different route, as shown in Reaction Scheme IV below:

REACTION SCHEME IV

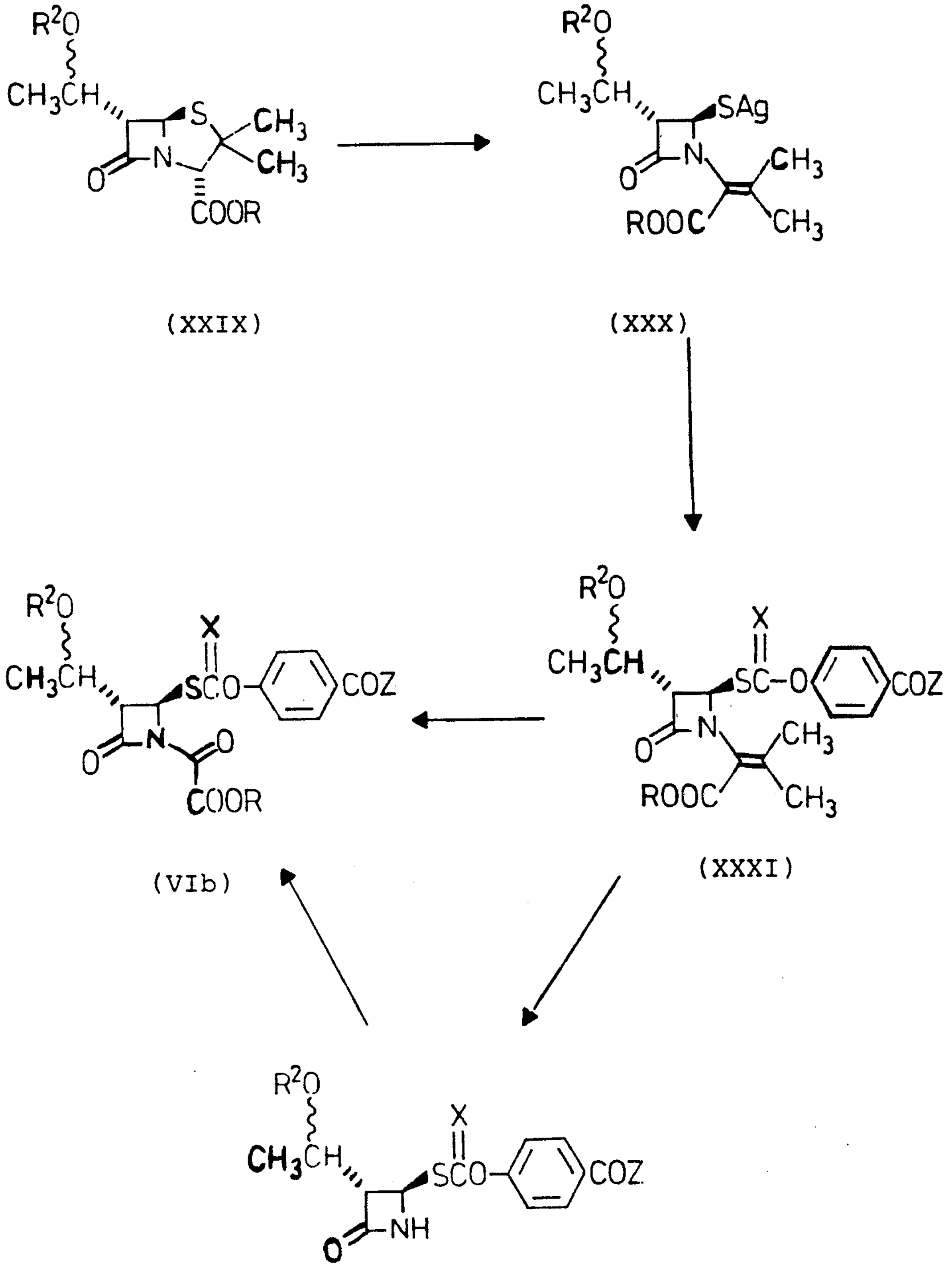

(XXIX)

(XXX)

(VIb)

(XXXI)

(XXII)

in which R, $R^2$, X and Z are as defined above.

The optionally protected penicillin derivative XXIX is reacted with a silver salt, for example, as described by M. Alpegiani et al, J. Am. Chem. Soc. 107, 6398 (1985). The reaction is carried out for example, using silver nitrate generally in the presence of a base, for example, DBN or DBU. The reaction is generally carried out in a solvent that is inert under the reaction conditions, for example, acetonitrile, and at a temperature within the range of from -30°C to +40°C, preferably from 0° to 20°C.

The resulting silver compound XXX may be isolated but generally the reaction mixture is treated with a compound of formula XIIa

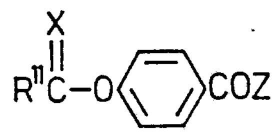

(XIIa)

in which $R^{11}$, X and Z are defined as above, optionally in the presence of a base, to give a compound of formula XXXI. The base, if present, is generally an organic base, especially triethylamine or pyridine. The reaction is generally carried out in a solvent, for example, an ether, for example, diethyl ether; a chlorinated hydrocarbon, for example, dichloromethane or chloroform; acetonitrile, dimethylformamide or ethyl acetate. The reaction is generally carried out at a temperature within the range of from -20° to +45°C, preferably from 0° to 20°C.

Compound XXXI may be converted directly into compound VIb by ozonolysis, or by using a chemical oxidising agent, for example, a permanganate, for example, potassium permanganate; or a persulphate, for

example, potassium persulphate.

The oxidation is carried out in a solvent or diluent that is inert under the reaction conditions, for example, a chlorinated hydrocarbon, for example, dichloromethane or chloroform or an oxygenated hydrocarbon, for example, diethyl ether, tetrahydrofuran or ethyl acetate, methanol or acetone. Particularly preferred solvents are dichloromethane, ethyl acetate and acetone.

The oxidation is generally carried out at a temperature within the range of from -78 to +20°C, preferably from -50 to 0°C.

Alternatively, compound XXXI may be converted into compound VIb via compound XXII. In this case, ozonolysis or chemical oxidation is carried out in the same manner and under the same conditions as described above for the production of compound VIb. The product from this reaction is then treated with methanol at room temperature, optionally in the presence of silica gel to give compound XXII. Compound VIb may then be obtained from compound XXII as described in Reaction Scheme III above.

In compound XXXI, when X represents a sulphur atom, the oxidation can be controlled to give a compound of formula VIb in which X represents either a sulphur or an oxygen atom. Those compounds of formula VIb in which X represents a sulphur atom, are preferred and such compounds may be obtained by using an appropriate predetermined amount of ozone or chemical oxidant.

A carboxy protecting group R in compound XXXI in this Reaction Scheme is that present in the starting penam XXIX. If this carboxy protecting group is a group desired for the final penem of formula I, for example, a <u>p</u>-nitrobenzyl group, then the direct route from

compound XXXI to compound VIb is preferred. If, however, it is desired to change the carboxy protecting group R, then the indirect route via compound XXII enables this to be done.

Any interconversion preferred or required, to produce a desired compound Ia may be carried out at an appropriate point, for example, as described briefly above and in more detail below.

The routes to compound I described in Reaction Scheme IV have the advantage of being shorter than would be described in UK Specification No. 2 161 161A, especially if the route chosen is from compound XXIX via compounds XXX, XXXI and VIb. Moreover, the starting material of formula XXIX is readily available and relatively cheap (the silver salt can be recovered for further use). Furthermore, the stereochemistry at position 5 of the starting material of formula XXIX is retained throughout the reaction sequence. The naturally occurring peniclin generally used has the desired 5R-stereochemistry, and so ultimately does the end product of formula Ia.

As indicated above, the present invention provides a process for the production of a compound of formula Ia

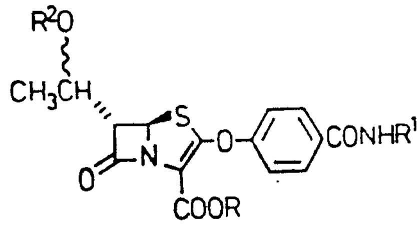

(Ia)

in which R, $R^1$ and $R^2$ are defined as above, which comprises

producing a compound of formula Ib

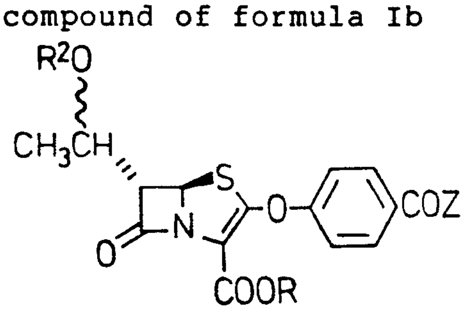

(Ib)

in which R, $R^2$ and Z are defined as above by cyclising a compound of formula VIIIa or VIIIb

(VIIIa)

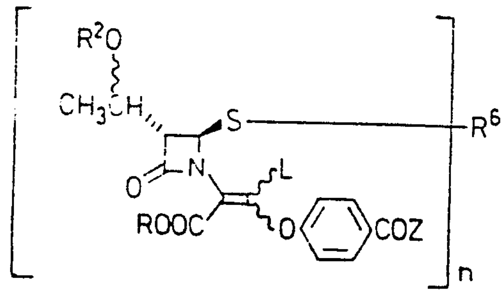

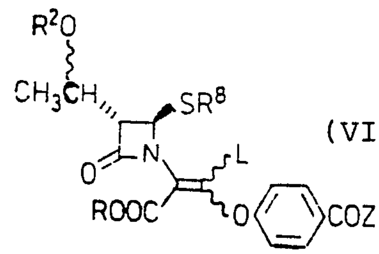

(VIIIb)

in which formulae R, $R^2$, $R^6$, and $R^8$, n, L and Z are defined as above and

(i) in the case of compound VIIIa, effecting cyclisation, and

(ii) in the case of compound VIIIb, removing the sulphur protecting group, if present, and either reacting the resulting thiol compound with a

reagent capable of converting that compound into a compound of formula VIIIa, and cyclising compound VIIIa or cyclising the thiol compound by treatment with a base; and

as required and as desired, carrying out any one or more of the reactions described in steps (2) to (4) above, in any appropriate order.

In some cases, on removing the sulphur protecting group $R^8$ and reacting compound VIIIb with a metal salt, compound Ib generally in protected form may be produced directly. In some cases, a compound of formula VIIIa is produced as an intermediate and may be isolated, if desired, or reacted further *in situ*.

Compound VIIIa can be cyclised spontaneously to give a compound of formula Ib. This reaction is preferably carried out in a solvent or diluent, that is inert under the reaction conditions, for example, acetonitrile, an aromatic hydrocarbon, for example, benzene or toluene, a halogenated hydrocarbon, for example, chloroform or dichloromethane, or an ether, for example, diethyl ether, dioxane or tetrahydrofuran. Acetonitrile is particularly preferred. The reaction mixture may be cooled, in which case the cyclisation takes place more slowly, or may be heated, in which case the reaction takes place more quickly. An example of a temperature range for carrying out this process is $0^{\circ}$ to $100^{\circ}C$.

As indicated above compound VIIIa may be produced from compound VIIIb and may be isolated before cyclisation, or compound VIIIa may be produced from compound VIIIb and cyclised *in situ*. In some cases, compound VIIIa may not be formed as an intermediate, but compound VIIIb may be converted directly to compound Ib.

Methods of removing sulphur protecting groups are

well known , for example, McOmie, Protecting Groups in Organic Chemistry Plenum Press, London and New York, 1981, and T.W. Greene, Protective Groups in Organic Synthesis, Wiley, New York 1981.

In some cases, it is not necessary to react compound VIIIb with a suitable metal salt as cyclisation occurs spontaneously on removal of the sulphur-protecting group or upon treatment with a base. This is the case, for example, when $R^8$ in compound VIIIb represents a p-nitrobenzyl group. This compound VIIIb may be reduced, for example, electrochemically or by catalytic hydrogenation. The use of, for example, hydrogen over a palladium-on-charcoal catalyst, followed by treatment with a base, for example, an organic base, for example, triethylamine yields compound Ib directly. A wide variety of bases may be used, providing the pK is greater than 9.

In some cases, $R^8$ must be removed first, and the resulting sulphur-deprotected compound reacted with a suitable metal salt, and in yet other cases the protected compound VIIIb need not be deprotected at the sulphur atom, but, can be reacted directly with a suitable metal salt.

A suitable metal salt is a salt capable of yielding Ag(I), Hg(II), Pb(II) or Cu(II), for example, silver nitrate, mercury diacetate, lead dinitrate, or copper (II) chloride.

When $R^8$ in compound VIIIb represents a triphenylmethyl group, then compound VIIIb may be reacted with a suitable metal salt, for example, with mercury diacetate or silver nitrate, especially silver nitrate. This reaction which is generally carried out in a solvent or diluent, for exmaple, acetonitrile, a halogenated hydrocarbon, for example, chloroform or

dichloromethane or an ether, for example, diethyl ether, tetrahydrofuran or dioxane, may be conducted in the presence of a protic or Lewis acid, for example, trifluoroacetic acid, anisole or hydrogen bromide in acetic acid. The reaction temperature is, for example, within the range of from -20 to +100$^{o}$C, preferably from 0 to +50$^{o}$C. Compound VIIIa may be isolated if desired by carrying out the reaction at a lower temperature, but it is generally satisfactory to use a temperature within the range of from 0 to 80$^{o}$C, whereupon the cyclisation occurs in situ.

When $R^8$ in compound VIIIb represents a propen-2-yl group, compound VIIIb may be converted into compound VIIIa by oxidation, for example, using ozone or a permanganate or periodate followed by hydrolytic cleavage using, for example, an organic base, for example, imidazole or pyridine in the presence of a metal salt capable of yielding Ag(I), Hg(II), Pb(II) or Cu(II), for example, as described above. The ozonolysis reaction is, for example, carried out at a temperature within the range of from -80 to 0$^{o}$C, preferably from -60 to -20$^{o}$C.

The hydrolytic cleavage may be carried out using, for example, silver nitrate or $CdCO_3$ in acetonitrile and in the presence of an acid binder. This reaction is preferably carried out at a temperature within the range of from -40 to +40$^{o}$C. The reaction is generally carried out in a solvent or diluent.

When $R^8$ in compound VIIIa represents a t-butyl group this compound may be reacted with, for example, hydrogen fluoride, in a solvent, for example, anisole.

The reaction product from any of the above reactions has the desired 5R-stereochemistry and, if desired or required, may be subjected to any of the further processes described above, for example, the

product may be deprotected to give a compound of formula I, or may be converted into another ester, for example, a pro-drug form, and/or salt form, for example, as described briefly above, and in more detail below.

A compound of formula VIIIb may be produced from a compound of formula XXXII

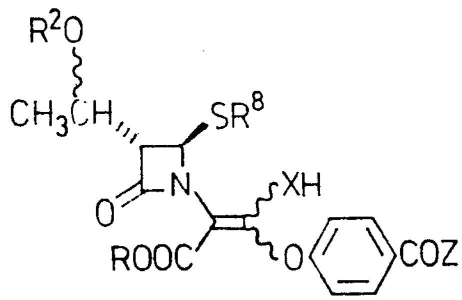

(XXXII)

in which R, $R^2$, $R^8$, X and Z are as defined above, by reaction with a reagent capable of introducing a group as defined above, for example, an acylating agent, for example, acetic anhydride or a trimethyl-carbonyl halide, or another activating group for example, $CH_3SO_2Cl$, $(CF_3SO_2)_2O$, $(CF_3CO)_2O$ or p-toluenesulphonyl chloride.

The reaction is generally carried out in the presence of a base, for example, an organic base having a pK $\geqslant$ 5, for example, a trialkylamine, for example, triethylamine, or pyridine. The reaction is generally carried out in a solvent, for example, a hydrocarbon, for example, benzene or toluene; an ether, for example, diethyl ether, tetrahydrofuran or dioxane; a halogenated hydrocarbon, for example, dichloromethane, or an ester, for example, ethyl acetate.

A compound of formula XXXII may be produced by reacting a compound of formula XXXIII

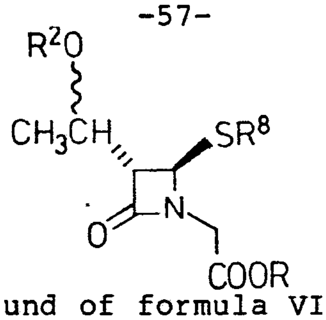

(XXXIII)

with a compound of formula VII

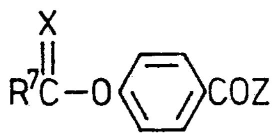

(VII)

in which Z and X are as defined above, $R^7$ represents a halogen atom, especially a chlorine atom, in the presence of a base.

While many phenyl chlorothionoformates are known, other chlorothionoformats of formula VII have not been described before; they may be prepared by methods analogous to those described for the preparation of known compounds, see for example, Rivier & Schalch, Helv. Chem. Acta., Vol. 6, 1923, p 605 and Reich and Martin, Chem. Berichte, Vol. 98, 1965, p 2063. Chloroformates of formula VII may be produced by reacting the appropriate phenol of formula IV

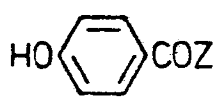

(IV)

in which Z is as defined above, with phosgene. The reaction is generally carried out in a solvent or diluent, for example, an aliphatic or aromatic hydrocarbon or an aliphatic or aromatic chlorinated hydrocarbon, for example, hexane, benzene, toluene carbon tetrachloride, methylene chloride. The reaction

temperature is, for example, within the range of from -20 to +20°C. For reactions of this type see for example, R.J. Cotter, M. Matzner and R.P. Kurkjy, Chem. in Industry 1965, 791 or D.R. Patent Specification No. 287 805.

The reaction between compound XXXIII and compound VII is carried out in the presence of a base, preferably having a $pK_a \geq 20$, preferably a metallated amine, and examples of preferred bases are lithium diisopropylamide, lithium hexamethyldisiazide, lithium 2,2,6,6-tetramethylpiperidide, lithium cyclohexyl isopropylamide, and sodamide.

The reaction is generally carried out in an aprotic solvent, for example, an oxygenated hydrocarbon, preferably an ether, for example, diethyl ether, tetrahydrofuran, dioxane, glyme or diglyme. The reaction temperature is, for example, from -120 to +30°C, preferably -78 to -20°C.

The amount of base used is, for example, from 1 to 3 moles, calculated per mole of compound VII, preferably from 1.5 to 2.5 moles of base. The compound of formula XXXIII is preferably used in an amount of from 1 to 1.5 moles per mole of compound VII, preferably from 1 to 1.1 moles of compound VII per mole of compound XXXIII.

The reaction may be carried out as follows: The base may be added to a stirred solution of compounds XXXIII and VII. Alternatively, to a stirred solution of compound XXXIII under an inert atmosphere is added the base and subsequently a solution of compound VII in the same or different solvent.

Some compounds of formula XXXIII are known, see, for example, GB 2 102 798A. Others may be prepared analogously.

An advantage of this process for the production of

a compound of formula I is that the stereochemistry at position 5 can be established in the initial starting material and maintained throughout the reactions involved. In particular, the initial starting material is conveniently compound XXXIII, which can be obtained from commercially available materials and which, being derived from naturally occuring pencillin, has the desired R-stereochemistry at position 4. As no high temperature need be used in the various reactions involved, for example, the cyclisation of VIIIa or VIIIb to compound Ib can occur spontaneously, there is no substantial thermal interconversion at position 5. Accordingly, by starting with a substantially pure 4R-azetidinone derivative, a substantially pure 5R-compound of formula I can be obtained.

In each of the processes described above for the production of a compound of formula Ia .

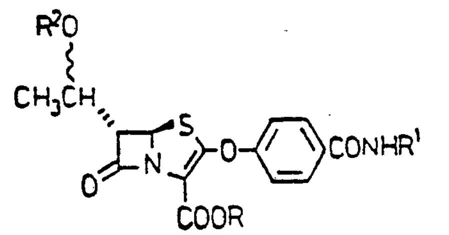

(Ia)

in which R, $R^1$ and $R^2$ are defined as above, the initial product has the formula Ib

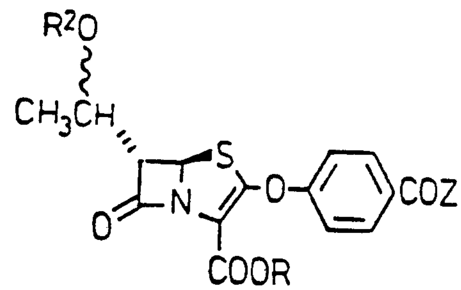

(Ib)

in which R, $R^2$ and Z are defined as above.

It will be appreciated that as mentioned above the group -COZ in formula Ib can represent the ultimately desired $-CONHR^1$ group. In this case, compound Ia is produced directly. If -COZ in compound Ib represents a protected aminocarbonyl group $-CONHR^3$, $-CONR^1R^3$ or $-CONR^3R^3$, $R^3$ representing a protecting group, the protecting group(s) $R^3$ may be removed after formation of compound Ib or at any earlier stage of the reaction sequence. Similarly, if -COZ represents a $-COR^4$ group, for example, a $-COXR^{5'}$ group (X, $R^4$ and $R^{5'}$ being defined as above), then this group may be converted to the desired aminocarbonyl group at any stage in the

reaction sequence, either before formation of compound Ib or after the otherwise desired compound is obtained.

Furthermore, and analogously, a -COOH group represented by -COZ may be converted into a free aminocarbonyl or lower alkylaminocarbonyl group at any stage before or after formation of compound Ib, for example, at any appropriate point in any of the reaction sequences involved in the production of compound Ib, for example, in compound II. It is generally necessary to activate this carboxy group before reaction with ammonia or a lower alkylamine, respectively, or with an equivalent reagent known in the art. The activating group may be introduced at any stage of the reaction sequence and, independently or concommitantly, the activated carboxyl group converted into a free aminocarbonyl or lower alkylaminocarbonyl group at any stage, that is to say, the intermediate activated compound may be produced in a separate reaction step and isolated, if desired, or it may be converted _in situ_ into an aminocarbonyl compound.

The conversion of a carboxylic acid group into a free aminocarbonyl or lower alkylaminocarbonyl group is well known in chemistry, and there is available to those versed in the art a wide range of reagents and methods. In general, as indicated above, the reagents function by converting a carboxylic acid group into an activated derivative thereof, which derivative is then reacted with ammonia or a lower alkylamine. Examples of the activation of a carboxylic acid group are by conversions as follows:

(i) to an activated ester, for example, to a phenyl ester using, for example, a bisphenyl carbonate;
(ii) to a phosphorous or phosphoric ester, or a phosphoric acid anhydride, using for example, a

phosphinyl halide or a phosphoryl halide;

(iii) to a carboxylic acid anhydride, especially a mixed anhydride, using for example, an acid chloride or bromide, for example, pivaloyl bromide, a carbodiimide, for example, dicyclohexylcarbodiimide or 1-(3-dimethylaminopropyl)-3-ethylcarbodiimide, or a chloroformate;

(iv) to an imidazolide using, for example, N,N'-carbonyldiimidazole;

(v) to an acid chloride using, for example, thionyl chloride; or

(vi) to an O-acylurea using, for example, a carbodiimide, for example, as described above, and if desired, converting the O-acylurea into an active ester, for example, an ester with 1-hydroxybenzotriazole or with N-hydroxysuccinimide.

Examples, of activated acid groups $-COR^4$ that can be converted into groups of formula $-CONHR^1$ under reaction conditions that do not affect other parts of the compounds in question (other than groups $-COXR^5$ as defined above) are, for example, groups of the formula $-COOR^{19}$ in which $R^{19}$ represents one of the following groups

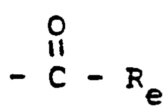
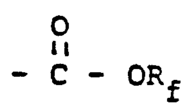
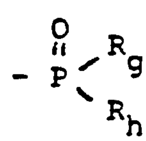
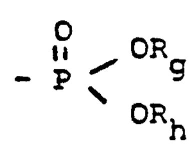

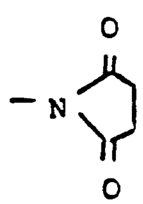
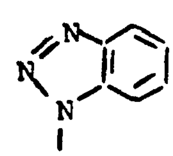

in which $R_e$ represents an alkyl group having up to 4 carbon atoms, especially a t-butyl group; $R_f$ represents an alkyl group having up to 4 carbon atoms, especially an iso-butyl group; $R_g$ and $R_h$, which may be the same or different, each represents an alkyl group having up to 4 carbon atoms or a phenyl group.

The above methods are examples of the techniques available to the art, and do not constitute an exhaustive list. For further information see, for example, M. Bodanszky, Y.S. Klausner and M.A. Ondetti, "Peptide Synthesis", J. Wiley and Sons, New York, 1976, and N.F. Norton, Organic Reactions, Vol. 12, 157 (1962).

Some of the methods for converting a carboxylic acid group into an aminocarbonyl or lower alkylaminocarbonyl group are extremely mild and therefore well suited to the conversion of a penem containing a carboxylic acid group into a penem containing an aminocarbonyl or lower alkylaminocarbonyl group without damage to any other part of the molecule. Thus, for example, a solution of a carboxylic acid of formula Ib (Z=OH) in an inert solvent for example, dichloromethane, acetonitrile or tetrahydrofuran, may be treated with a carbodiimide, for example, dicyclohexylcarbodiimide and 1-hydroxybenzotriazole at a temperature within the range of from -40 to +40°C, preferably from 0 to 20°C, to form the benzotriazol-1-yl ester.

In the case of a compound in which -COZ represents the group $-COXR^{5'}$, $R^{5'}$ preferably represents a phenyl group substituted by one or more chlorine and/or fluorine atoms. When X represents a sulphur or oxygen atom, $R^{5'}$ especially represents a pentafluorophenyl group, and when X represents a sulphur atom, 4-chlorophenyl, 4-cyanophenyl, 4-methoxyphenyl and 2,4,5-trichlorophenyl groups are also particularly preferred

as $R^{5'}$.

Reaction of a compound having an activated acid group $-COR^4$ with ammonia or a lower alkylamine is preferably carried out at a temperature within the range of from -40 to +40°C, preferably from 0 to 20°C. The choice of solvent is wide, provided that the solvent does not itself react with any of the reagents or intermediates. For this reason it is often preferable to use a solvent mixture that is substantially free of water. Example of suitable solvents are dimethylformamide and acetonitrile.

The reaction between ammonia or a lower alkylamine and the appropriate compound may be carried out in the presence of a metal salt, especially a salt of a metal selected from Groups IB, IIB and VIII of the Periodic Classification of the Elements (cf E. Cartmell & G.W.A. Fowles, Valency and Molecular Structure, Butterworths, 1966), for example, a salt or copper, rhodium, mercury, zinc, cadmium, or especially, silver. The salt is, for example, a salt with an organic or inorganic acid, for example, with perchloric tetrafluoroboric, acetic, trifluoromethanesulphonic, or trifluoroacetic acid, or with imidazole. Examples of preferred salts are silver acetate, silver trifluoroacetate, silver trifluoromethanesulphonate, and silver imidazolide.

The degree of advantage resulting from the presence of a metal salt during the reaction between ammonia or a lower alkylamine and the appropriate compound depends on the reactivity of the $-COR^4$ group in the compound in question, and may be determined empirically, for example, there is not generally a substantial advantage when $-COR^4$ represents a group $-COXR^{5'}$ in which X represents an oxygen atom. In the case of a compound having a pentafluorophenylthio

ester group, a metal salt may be used if desired, but the resulting advantage is not large, whereas for other compounds in which X represents a sulphur atom, the presence of a metal salt during the reaction may greatly increase the yield.

A cyano group COZ can be converted into a group $-NHR^1$ by methods as described in J. March, Advanced Organic Chemistry, 3rd Edition, J. Wiley. (1984), 788-789.

A further reaction which may be carried out at any suitable point in a reaction sequence before or after formation of a compound Ib or Ia is the conversion of an 8S-hydroxy group, which may be free or protected, into the desired 8R-configuration. Methods for carrying out a stereochemical inversion at the 8-position of a penem are known, see, for example, I. Shinkai et al, Tetrahedron Letters, 1982, 4899.

In one example of such a technique a free 8-hydroxy group is oxidised to give an oxo group, which is then reduced to a hydroxy group. The oxidation may be carried out using, for example, a dimethylsulphoxide-trifluoroacetic anhydride-triethylamine reagent, cf A.K. Sharma and D. Swern, Tetrahedron Letters, 1974, 1508, and S.L. Huang et al, Synthesis 1983, 297. The reducing agent used may be an alkali metal tri-sec-butylborohydride in which the alkali metal component is, for example, lithium, sodium or potassium, c.f. F.A., Bouffard et al, J. Org. Chem. 1981, 46, 2210, or may be a di-isopropylamine-borane complex in the presence of magnesium trifluoroacetate cf P.J. Reider et al, Tetrahedron Letters 23 2293 (1982). The reduction may give one substantially pure diastereoisomer, or a mixture of isomers which can be separated.

In another method, a di-lower alkyl

azodicarboxylate-triphenylphosphine complex reagent is used. This type of reaction is described by O. Mitsonobu in Synthesis, 1981 page 1. It involves, for example, reacting the free hydroxy compound with a mixture of triphenylphosphine and the di-lower alkyl azodicarboxylate in the presence of a nucleophilic agent, for example, an acid, for example, a carboxylic acid, for example, formic or benzoic acid, or a carboxylate and in solution in a solvent that is inert to the reaction conditions followed by hydrolysis of the modified hydroxy group to give the free hydroxy group again, for example, as shown below in the case of a hydroxy group converted into a benzoyloxy group:

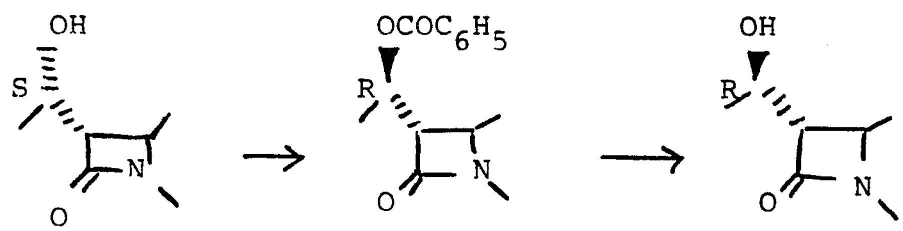

The term "known" is used herein to mean in actual use in the art or described in the literature of the art. The term "lower" denotes a group, radical or moiety having from 1 to 4 carbon atoms.

The various 2-ester, 8-ester and salt interconversions can also be carried out at any appropriate point in a reaction scheme, after formation of compound Ib or Ia or before their formation, that is to say, at any appropriate point in the various reaction sequences described above for each of the process variants.

In general, when manipulations are to be carried out on a compound then conventional protecting groups

at positions 2 and 8 may be advisable to prevent those groups from taking part in undesired reactions.

Preferred hydroxy-protecting groups $R^1$ are those that may be removed under conditions that do not otherwise affect the compound in which they are present.

One type of preferred hydroxy protecting group $R^2$ can be removed under acidic conditions. Such groups are well known in the art and are, for example, tetrahydropyranyl and tetrahydrofuranyl groups; acetal and ketal groups, for example, of formula

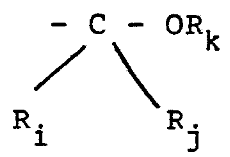

in which $R_i$ and $R_j$ which may be the same or different, each represents a hydrogen atom or a lower alkyl group, preferably a methyl group, or $R_i$ and $R_j$ together with the carbon atoms to which they are attached, represent a cycloalkyl ring having from 4 to 7 carbon atoms, and $R_k$ represents a lower alkyl group, preferably a methyl or ethyl group, or $R_i$ and $R_k$, together with the carbon atom and the oxygen atom to which they are attached, respectively, represent a tetrahydropyranyl ring and, in this case, $R_j$ is hydrogen; also silyl ethers, for example, having three substituents on the silicon atom, and preferably up to 24 carbon atoms in total, the three substituents being the same or different, and selected from alkyl, alkenyl and cycloalkyl groups, and phenyl and phenalkyl groups which may be unsubstituted or substituted as defined above, for example, $-SiR_lR_mR_n$ groups, in which $R_l$, $R_m$ and $R_n$ may be the same or different, and each represents a lower alkyl group or a phenyl group,

for example, giving trimethylsilyl, triethylsilyl, diphenyl-t-butylsilyl, t-butyldimethylsilyl, and methyldiphenylsilyl groups; and stannyl groups, for example, having up to 24 carbon atoms and three substituents, which may be the same or different, selected from alkyl, alkenyl, cycloaklyl, alkoxy and phenoxy groups, and phenyl and phenalkyl groups which may be unsubstituted or substituted, for example, groups of the formula $SnR_oR_pR_q$, in which $R_o$, $R_p$ and $R_q$, which may be the same or different, each represents a lower alkyl group, for example, a tri-n-butylstannyl group. (The term "lower" is used in the present specification to denote groups having up to 4 carbon atoms.)

A further type of preferred hydroxy protecting groups $R^2$ are carbonates, for example, unusubstituted or substituted alkyl or phenylalkyl carbonates, for example, p-nitrobenzyl carbonate and trichloroethyl carbonate.

Preferred hydroxy protecting groups $R^2$ are p-nitrobenzyl carbonate, trichloroethyl carbonate, tetrahydropyranyl, 2-methoxyprop-2-yl, trimethylsilyl and, especially, triethylsilyl and t-butyldimethylsilyl groups.

Such groups may be removed, when required, by acid hydrolysis, for example, using 0.1 to 2M, preferably 0.5M hydrochloric acid, for example, 6M HCl in, for example, tetrahydrofuran, cf. Belgian patent specification No. 882 764; or aqueous hydrogen fluoride, for example, in the presence of acetonitrile, cf. J. Chem. Soc. Perkin 1, 1981, 2055.

In a compound of formula Ia, the 8-hydroxy group, if esterified, is preferably in "pro-drug" form that is to say, is esterified with a group that can be removed in vivo to give the free hydroxy group, that is to

say, an ester group that can be removed under physiological conditions. Examples of suitable esterifying groups are carboxylic acid acyl groups of the formula $R^{20}CO-$ in which $R^{20}$ represents a hydrogen atom or a straight or branched chain alkyl group having from 1 to 6 carbon atoms, especially a methyl, ethyl or t-butyl group, or represents a phenyl group or a phenoxyalkyl group in which the alkyl moiety is straight-chained or branched and has from 1 to 4 carbon atoms, and is especially a methylene group.

A non-physiologically removable protecting group $R^2$ is generally removed from a resulting compound of formula Ia, and may be replaced by a physiologically removable group, if desired. In some cases, a carboxylic acid acyl group $R^2$ may fulfil a protective role during the synthesis of compound Ia. Such a dual function protective group may be removed, retained or replaced in formula Ia, as desired.

An ester group at the 8-position may be the only ester group present, or it may be present in addition to an ester group at the 2-carboxyl group. As mentioned above, a physiologically removable group may be present as a protecting group $R^2$ during the formation of a compound of formula Ia, or it may be introduced at the free 8-hydroxy group of a compound of formula Ia, after removal of a non-physiologically removable hydroxy protecting group, if present. An esterifying group may be introduced at the 8-hydroxy group by a reaction with an organic acid derivative in known manner. A particularly convenient method is to react a compound of formula Ia with an activated acid derivative, for example, an acid anhydride in the presence of an organic base, for example, 4-dimethylaminopyridine.

As indicated above, various compounds may be in the form of an ester -COOR at the carboxy group

(R representing a carboxy esterifying group). Such an ester is particularly one that can be converted into the free acid by hydrolysis, photolysis, or reduction. Examples of such esters are those formed with unsubstituted or substituted aliphatic alcohols or phenols having up to 20 carbon atoms in total. In an esterified carboxy group of formula $-COOR_x$ the group $R_x$ may be, for example, a straight or branched chain substituted or unsubstituted alkyl, alkenyl or alkynyl group having up to 18 carbon atoms, preferably up to 8 carbon atoms, and especially up to 6 carbon atoms, for example, a methyl, ethyl, n-propyl, iso-propyl, n-butyl, sec-butyl, iso-butyl, tert-butyl, n-pentyl, n-hexyl, allyl or vinyl group. An aliphatic group $R_x$ especially a methyl or ethyl group, may be substituted, for example, by an acyloxy group (further details of such groups are given below) or by an aminoalkanoyloxy group, or an ethyl group $R_x$ may be substituted by an optionally substituted amino group at the 2-position, or a methyl group $R_x$ may be substituted by phenyl groups. A phenyl group, either *per se* or as a substituent of an aliphatic group, may be substituted, for example, by one or more substituents, selected especially from nitro groups and halogen atoms. Examples of phenyl substituted-aliphatic groups are benzyl, p-nitro-benzyl, benzhydryl and trityl groups.

As indicated above, for pharmaceutical use, an ester group is especially one that can be removed by hydrolysis, photolysis or reduction, or two or more of these methods may be used, for example, reduction followed by hydrolysis. A group $R_x$ that can be removed readily without substantial degradation of the rest of the molecule is particularly useful as a carboxy protecting group. Examples of esters that are

readily split by reduction are phenyl substituted-methyl esters, which may be unsubstituted or substituted, for example, benzyl, p-nitrobenzyl, benzhydryl and trityl esters.

Reduction of an ester, for example, a phenyl substituted-methyl ester, for example, a p-nitro-benzyl ester, may be carried out using hydrogen and a metal catalyst, for example, a noble metal catalyst, for example, platinum, palladium or rhodium, which catalyst may be supported, for example, on charcoal or kieselguhr.

Alternatively, a p-nitrobenzyl ester may be converted into the corresponding free acid by a two-step method, with an initial reduction of the nitro group followed by hydrolysis. The nitro group may be reduced by noble method catalysed hydrogenation, for example, using platinum, or palladium on carbon, or by a metal reducing agent, for example, zinc in acetic acid. Other metal reducing agents are, for example, aluminium amalgam, and iron and ammonium chloride, see for example, British Patent Specification No. 1,582,960. Reduction of the nitro group is followed by hydrolysis which may occur *in situ* during reduction of the nitro group or which may be carried out subsequently by treatment with an acid or a base.

An o-nitrobenzyl ester may be split off by photolysis, or a group may be used which is capable of removal by base hydrolysis, for example, acetylmethyl and acetoxymethyl ester groups.

There may be used an esterifying group that is removable under physiological conditions, that is to say, the esterifying group is split off *in vivo* to give the free acid or the carboxylate, for example, an acyloxymethyl or acyloxyethyl ester, for example, an acetoxymethyl, 1'-(acetoxy)ethyl or pivaloyloxymethyl

ester, a 5-methyldioxalen-2-on-4-yl-methyl ester, an aminoalkanoyloxymethyl ester, for example, a glycyloxymethyl, L-valyloxymethyl or L-leucyloxymethyl ester or a phthalidyl ester, or a 1'-(alkoxycarbonyloxy)ethyl ester, for example, a 1'-(methoxycarbonyloxy)-ethyl or 1'-(ethoxycarbonyloxy)ethyl ester, or an optionally substituted 2-aminoethyl ester, for example, a 2-diethylaminoethyl or 2-(1-morpholino)-ethyl ester (acyl and alkanoyl groups having 1 to 12 carbon atoms).

Preferred esterifying groups are p-nitrobenzyl groups for protection, and phthalidyl, pivaloyloxymethyl, ethoxycarbonyloxymethyl, and 5-methyldioxalen-2-on-4-yl-methyl groups as "pro-drug" groups.

For pharmaceutical use, it is preferable that the 8-hydroxy group in compound Ia should either be free or in "pro-drug" form, that is to say, in the form of an ester that can be hydrolysed in vivo to give the free hydroxy group. Similarly, the 2-carboxy group may be free, in "pro-drug" form, or in physiologically tolerable salt form.

The present invention also provides salts of those compounds of formula Ia that have salt-forming groups, especially the salts of a free acid of formula I and acid addition salts of compounds of formula Ia having a basic group. The salts are especially physiologically tolerable salts, for example, alkali metal and alkaline earth metal salts, for example, sodium, potassium, lithium, calcium, and magnesium salts, ammonium salts, and salts with organic amines; also physiologically tolerable acid addition salts.

These may be formed with a suitable inorganic or organic acid, for example, hydrochloric acid, sulphuric acid, or an organic carboxylic or organic sulphonic acid, for example, p-toluenesulphonic acid.

A salt of a free acid of formula Ia may be

produced by reacting the free acid with the appropriate base in a solvent, preferably under those conditions under which the salt precipitates. A preferred base is potassium 2-ethylhexanoate.

A salt may be produced directly from an ester by splitting off the ester group under suitable reaction conditions, for example, catalytic reduction of an ester, for example, a p-nitrobenzyl ester, in an aqueous/organic solvent, for example, comprising water and ethyl acetate, dioxane or tetrahydrofuran, in the presence of a metal salt, especially a metal bicarbonate, for example, in an equivalent amount or in a slight excess, yields the salt directly.

When an acidic centre and a basic centre are both present in a compound of formula Ia, the compound may exist in zwitterionic form.

Protecting groups may be introduced or removed at any appropriate point in the reactions involved in the production of a compound of formula Ia.

At any stage in the production of a compound of formula Ia, a compound produced may be isolated from the reaction mixture in which it was prepared and, if desired, purified by the appropriate techniques used for the purification of organic compounds, for example, chromatography and crystallisation.

As indicated above, various intermediates may be produced in the form of mixtures of isomers of various kinds. Such mixtures may be separated or resolved at any stage, or an isomeric mixture may be used *per se* for subsequent reactions.

Compounds of formula Ia possess excellent activity against gram positive bacteria and gram negative bacteria. Moreover, the compounds of formula Ia and salts of show activity against these organisms in the presence of β-lactamase enzymes produced by both gram positive organisms, for example, *Staphylococcus*

aureus and gram negative organisism, for example, Enterobacter cloacae, thus indicating resistance to these enzymes.

Compounds of formula Ia are also inhibitors of β-lactamase enzymes.

The compounds of formula Ia and physiologically tolerable salts thereof may be used in humans and other animals to treat, for example, bacterial infections caused by both gram positive and gram negative bacteria, for example, Staphylococcus aureus, Streptococus pyogenes, Escherichia coli, Bacillus subtilis and Proteus morganii, some strains of which are resistant to conventional penicillin therapy.

Compounds of formula Ia are also particularly useful in combination with other β-lactam antibotics, in particular cephalosporins. In such a combination, very broad spectrum antibiotic activity is produced and synergystic effects are observed.

The present invention accordingly provides a pharmaceutical preparation which comprises a compound of formula Ia, or a physiologically tolerable salt thereof, or a mixture of two or more such substances as active ingredient, as produced according to the invention, in admixture or conjunction with a pharmaceutically suitable carrier. The preparation may also advantageously comprise one or more other pharmaceutically active substances, for example, another antibacterial substance, preferably one having a β-lactam ring and especially a cephaloporin antibiotic. The preparations may be in a form suitable for enteral or parenteral administration, for example, for oral, intravenous or intramuscular administration, for example, as tablets, capsules, syrups, or sterile injectable or infusion solutions. The preparations are advantageously in unit dosage form and preferably

comprise from 10 to 2000 mg of the active ingredient per unit dose. The daily dosage of the active ingredient is generally from 20 to 8000 mg, in divided doses, generally up to 4 doses.

The invention also provides the use of a compound of formula Ia or a physiologically tolerable ester or salt thereof as produced according to the present invention, for the manufacture of a medicament for the treatment of bacterial infections.

The invention further provides a method of treating mammals, especially humans, to combat a bacterial infection, which comprises administering to the mammal a compound of formula Ia or a physiologically tolerable ester or salt thereof, as produced according to the present invention. A β-lactam antibiotic, especially a cephalosporin antibiotic, is preferably also administered simultaneously or substantially simultaneously, by the same route or by a different route.

The invention further provides a pharmaceutical preparation which comprises an active ingredient as defined above, in unit dosage form.

The invention also provides a pharmaceutical preparation which comprises an active ingredient as defined above, or a physiologically tolerable salt thereof or a mixture of two or more such substances, in unit dosage form. Unit dosages are preferably as described above.

Compounds of formula Ia are also useful in the production of the antibacterially active compounds.

The present invention further provides compounds of formula Ia, Va, Vb, Vc, VIa, VIb, VIc, VId, VIIIa and VIIIb.

In the following Examples, which illustrate the invention, temperatures are expressed in degrees Celsius. Hyflo and Nujol are Registered Trade Marks, and t.l.c. denotes thin layer chromatography.

Example 1

4-Nitrobenzyl 2-{3(S)-[1(R)-(tert-butyldimethylsilyloxy)-ethyl]-4(R)-ethylthio azetidin-2-on-1-yl}-3-[4-(pentafluorophenoxycarbonyl)phenoxy]-3-trimethylacetylthio-propenoate

To a stirred mixture of 4-nitrobenzyl {3(S)-[1(R)-(tert-butyldimethylsilyloxy)-ethyl]-4(R)-ethylthio azetidin-2-on-1-yl}acetate (73.8 g), pentafluorophenyl 4-(chloro-thiocarbonyloxy)benzoate (64.1 g) and dry tetrahydrofuran (400 ml) was added at -78° a mixture of hexamethyldisilazane (75.5 ml) and tetrahydrofuran (200 ml) to which had been added at -20° a 1.55M solution of N-butyllithium in hexane (221 ml). After the resultant mixture had been stirred an additional 5 min. trimethylacetyl bromide (40.5 ml) was added; the temperature of the mixture was adjusted to -20° and the mixture was then partitioned between diethyl ether (2 l) and 0.33M hydrochloric acid (1 l). The aqueous layer was washed with diethyl ether, and the combined organic layers were washed with water, with saturated aqueous potassium bicarbonate, with brine, were dried over anhydrous magnesium sulphate and then evaporated in vacuo to afford the title compound as a viscous brown/red oil (150 g) as a mixture of E- and Z- isomers.

$\nu_{max}$ ($CDCl_3$) 1760 $cm^{-1}$
$\delta$ ($CDCl_3$) 8.23, 8.16, 7.56 and 7.17 (8H, 2 x AA'BB', J=8Hz); 5.44-5.24 (3H, m); 4.23 (1H, m); 3.23 (1H, ~t, J=3Hz); 2.81-2.58 (2H, m); 1.24 and 1.30 (3H, 2d, J=6Hz); 1.22 (3H, t, J=7Hz); 1.14 and 1.07 (9H, 2s); 0.87 and 0.79 (9H, 2s); 0.05 and 0.01 (6H, 2s) p.p.m.

BAD ORIGINAL

Example 2

4-Nitrobenzyl 2-{3(S)-[1(R)-(tert-butyldimethylsilyloxy)-ethyl]-4(R)-chloro-azetidin-2-on-1-yl}-3[4-(pentafluorophenoxycarbonyl)phenoxy]-3-trimethylacetyl-thiopropenoate

To a stirred solution of the unpurified product (150 g) from the preceding Example in dry chloroform (450 ml) at -45° was added a solution of chlorine (15.1 g) in carbon tetrachloride (226 ml); over a 45 min. period the mixture is warmed to 20°, and then evaporated *in vacuo* to afford the title compound (150 g) as a brown foam as a mixture of E and Z isomers.

$\nu_{max}$ ($CDCl_3$) 1790, 1771 and 1750 $cm^{-1}$;

$\delta$($CDCl_3$) 8.24, 8.17, 7.58 and 7.16 (8H, 2 x AA'BB', J=8.7Hz); 6.11, 6.03 (1H,2d, J=1.5Hz); 5.33 (2H,AB, J=14 Hz); 4.22 (1H, m); 3.48, 3.47 (1H, 2xdd, J=3.4 and 1.5Hz); 1.30, 1.24 (3H, 2xd, J=6Hz); 1.15, 1.08 (9H, 2xs); 0.86, 0.81 (9H, 2xs); 0.05, 0.02 (6H, 2xs) p.p.m.

Example 3

4-Nitrobenzyl 5(S),6(S)-[1(R)-(tert-butyldimethylsilyloxy)ethyl]-7-oxo-3-[4-(pentafluorophenoxycarbonyl)-phenoxy]-4-thia-1-azabicyclo-[3.2.0]hept-2-ene-2-carboxylate

To a stirred mixture of the unpurified product from Example 2 (150 g), tetrahydrofuran (270 ml) and water (30 ml) was added at 0° pyridine (32 ml); the reaction mixture was warmed to room temperature, was stirred for up to five hours, and then partitioned between diethyl ether and 0.5M-hydrochloric acid. The aqueous layer was washed with diethyl ether; the combined organic layers were washed with water, with saturated aqueous potassium bicarbonate and with brine, were dried over anhydrous magnesium sulphate, and evaporated *in vacuo* to afford the title compound as a brown foam (120 g). (This material was not purified before further reaction).

$\nu$ max ($CDCl_3$) 1790, 1761 and 1720 $cm^{-1}$;

$\delta$($CDCl_3$); 8.22, 8.18, 7.51, 7.26 (8H, 2xAA'BB', J=8.7Hz); 5.80 (1H, d, J=4.2Hz); 5.39, 5.20 (2H, AB, J=14Hz); 4.43 (1H,m); 3.99 (1H, dd, J=4.2 and 9.8Hz); 1.46 (3H, d, J=6Hz); 0.82 (9H,s) 0.06, 0.01 (6H, 2s). p.p.m.

Small (typically < 10%) amounts of the corresponding 5(R),6(S) penem carboxylate are also formed with physical data essentially as above except $\delta$ 5.70 (1H, d, J=1.6Hz) and 1.24 (3H, d, J=6Hz).

Example 4

4-Nitrobenzyl 5(S),6(S)-[1(R)-hydroxyethyl]-7-oxo-3-[4-(pentafluorophenoxycarbonyl)phenoxy]-4-thia-1-azabicyclo[3.2.0]hept-2-ene-2-carboxylate

To a stirred solution of unpurified 4-nitrobenzyl 5(S), 6(S)-[1R-(tert-butyldimethylsilyoxy)ethyl]-7-oxo-3-[4-(pentafluorophenoxycarbonyl)phenoxy]-4-thia-1-azabicyclo[3.2.0]hept-2-ene-2-carboxylate (7.35 g) in acetonitrile (250 ml) was added 40% aqueous hydrofluoric acid (80 ml). After 40 min., dichloromethane was added; the pH was adjusted to 5 with potassium bicarbonate and the mixture partitioned. The aqueous layer was washed with dichloromethane, and the combined organic extracts were washed with water and dried over anhydrous magnesium sulphate. Evaporation *in vacuo* afforded the title compound (6.06 g).

δ ($CDCl_3$) 8.23, 8.20, 7.53 and 7.29 (8H, 2 x AA'BB', J=8.8Hz); 5.84 (1H, d, J=4.1Hz); 5.41, 5.22 (2H, AB, J=13.7Hz); 4.44 (1H,m); 3.96 (1H, dd, J=4.1 and 10.3Hz); 1.53 (3H, d, J=6.1Hz) p.p.m.

Together with this compound there was also obtained a minor quantity (typically <10%) of the corresponding 5(R),6(S) penem compound whose physical properties are essentially as above except

δ 5.74 (1H, d, J=1.4Hz; 3.85 (1H, dd, J=1.4 and 6.5Hz); 1.39 (3H, d, J=6.3Hz) p.p.m.

Example 5

4-Nitrobenzyl 5(S),3-(4-aminocarbonylphenoxy)-6(S)-[1(R)-(tert-butyldimethylsilyloxy)ethyl]-7-oxo-4-thia-1-azabicyclo[3.2.0]hept-2-ene-2-carboxylate

Diisopropylethylamine (52 ml) and a solution of ammonia (5.13 g) in ethanol (114 ml) were added sequentially to a stirred solution of unpurified 4-nitrobenzyl 5(S), 6(S)-[1(R)-(tert-butyldimethylsilyloxy)ethyl]-7-oxo-3-[4-(pentafluorophenoxycarbonyl)phenoxy]-4-thia-1-azabicyclo-[3.2.0]hept-2-ene-2-carboxylate (120 g) in acetonitrile (600 ml). When t.l.c. ($SiO_2$, ethyl acetate) showed the reaction to be complete, ethyl acetate was added, and then the pH was adjusted to 2 with M-hydrochloric acid. After addition of water the mixture was partitioned; the aqueous layer was extracted with ethyl acetate, and the combined organic phases were washed with water, with 20% aqueous potassium carbonate, and with water, were dried and evaporated *in vacuo* to afford the crude title compound as a brown oil (~ 120 g). Non-penem byproducts could be removed by chromatography over silica gel of a portion of the residue; elution with ethyl acetate - hexane mixtures afforded a sample of a mixture of the pure title compound.

$\nu$ max (nujol) 1784, 1700, 1656 $cm^{-1}$;

$\delta$ ($CDCl_3$) 8.14, 7.48 (4H, AA'BB', J=8.8Hz); 7.83, 7.17 (4H, AA'BB', J=8.9Hz); 6.35 (2H, broad); 5.73 (1H, d, J=4.0Hz); 5.37, 5.18 (2H, AB, J=13.8Hz); 4.39 (1H, dq, J=9.8 and 6.0Hz); 3.93 (1H, dd, J=9.8 and

4.0Hz); 1.42 (3H, d, J=6.0Hz); 0.78 (9H,s); 0.06, 0.00 (6H,2s) p.p.m.

together with some of the corresponding 5(R),6(S) penem whose physical properties were essentially as above except for δ 5.65 (1H, d, J=1.4Hz); 4.25 (1H, m), 3.75 (1H, dd, J=1.4 and 4.8 Hz) and 1.25 (3H, d, J=6Hz) p.p.m.

Example 6

4-Nitrobenzyl 5(S),3-(4-aminocarbonylphenoxy)-6(S)-[1(R)-hydroxyethyl]-7-oxo-4-thia-1-azabicyclo-[3.2.0]hept-2-ene-2-carboxylate

Step A

To a stirred mixture of unpurified 4-nitrobenzyl 5(R),3-(4-aminocarbonylphenoxy)-6(S)-[1(R)-(tert-butyldimethylsilyloxy)ethyl]-7-oxo-4-thia-1-azabicyclo[3.2.0]hept-2-ene-2-carboxylate (136 g) in dry tetrahydrofuran (1500 ml) at 10° was added glacial acetic acid (86 ml), followed by a 1M solution of tetrabutylammonium fluoride in tetrahydrofuran (450 ml). The mixture was stirred at 20° for 16 hours, and was then worked up by partitioning between ethyl acetate and water. The aqueous layer was extracted with ethyl acetate, and the combined organic phases were washed with water, with aqueous sodium bicarbonate solution, with water and with brine and were evaporated in vacuo to afford a dark brown gum (ca 75 g). Diethyl ether (300 ml) was added; the mixture was stirred at room temperature for 2 hours and filtered to afford the

crude title compound (44 g) as a buff solid, which was chromatographed on silica gel. Elution with hexane - ethyl acetate - acetonitrile mixtures afforded the title compound (23 g) as a fawn solid.

m.p. 170-172$^o$ (dec)

$\nu$ max (Nujol) 3500, 3400, 3300, 1768, 1688, 1656, 1614 and 1606 $cm^{-1}$;

$\delta$ (DMSO-$d_6$) 8.19, 7.94, 7.60, 7.37 (8H, 2xAA'BB', J=8.8Hz); 8.03 (1H, broad); 7.44 (1H, broad); 5.90 (1H, d, J=3.9Hz) 5.40, 5.28 (2H, AB, J=14.2Hz); 5.20 (1H, d, J=5.1Hz); 4.11 (1H, m); 4.01 (1H, dd, J=10.3 and 3.9Hz); 1.29 (3H, d, J=5.8Hz) p.p.m.

Step A

To a solution of pure 4-nitrobenzyl 5(S),3-(4-aminocarbonylphenoxy)-6(S)-[1(R)-(tert-butyldimethylsilyloxy)ethyl]-7-oxo-4-thia-1-azabicyclo[3.2.0]hept-2-ene-2-carboxylate (37 g) in tetrahydrofuran (400 ml) at room temperature was added glacial acetic acid (35.5 ml) followed by a 1M solution of tetrabutylammonium fluoride in tetrahydrofuran (186 ml). The mixture was stirred for 16 hours and then diluted with ethyl acetate, and worked up as in Method A to afford the title compound (18 g) with physical properties identical to those given in Method A.

Step B

To a stirred mixture of the unpurified 4-nitrobenzyl 5(S),6(S)-[1(R)-hydroxyethyl]-7-oxo-3-[4-(penta-

fluorophenoxycarbonyl)phenoxy]-4-thia-1-azabicyclo-[3.2.0]hept-2-ene-2-carboxylate (5 g) obtained in Example 4 and acetonitrile (50 ml) at 0° was added diisopropylethylamine (2.24 ml) followed by a solution of ammonia (219 mg) in ethanol (6.7 ml). The mixture was warmed to 10° and stirred for one hour, and then partitioned between cold ethyl acetate and 0.25M hydrochloric acid. The aqueous phase was extracted with ethyl acetate, and the combined organic phases were washed with water, were dried over anhydrous magnesium sulphate and evaporated *in vacuo*. Chromatography of the residue over silica gel and elution with ethyl acetate - hexane mixtures afforded the title compound (1.2 g) with physical properties identical to those described in Method A.

Example 7

4-Nitrobenzyl 5(R), 3-(4-aminocarbonylphenoxy)-6(S)-[1(R)-hydroxyethyl]-7-oxo-4-thia-1-azabicyclo-[3.2.0]hept-2-ene-2-carboxylate

A stirred suspension of 4-nitrobenzyl 5(S), 3-[4-(aminocarbonyl)phenoxy]-6(S)-[1(R)-hydroxyethyl]-7-oxo-4-thia-1-azabicyclo[3.2.0]-hept-2-ene-2-carboxylate (18 g, but including ca 10% of the diastereoisomeric 5(R) compound) in acetonitrile (360 ml) was rapidly heated to 80° and then maintained at this temperature for 2 hours. The mixture was cooled to 20° over a 15 minute period and then allowed to stand at 0° for 30 minutes. Filtration, and washing of the residue with ethyl acetate - dichloromethane mixtures, and drying *in*

*vacuo* afforded pure title compound (14.5 g) as an off-white microcrystalline solid.

m.p. 196-200$^o$

$\nu$ max (Nujol) 3450(m), 3200, 1748(s), 1706(s) 1658, 1624 $cm^{-1}$.

$\delta$ (DMSO-$d_6$) 8.18, 7.58 (4H, AA'BB', J=8.7Hz); 7.92, 7.35 (4H, AA'BB', J=8.8Hz); 8.02 (1H, broad); 5.78 (1H, d, J=1.4Hz); 5.40, 5.27(2H, AB, J=14.3Hz); 5.25 (1H, broad; 4.04 (1H, m); 3.90 (1H, dd, J=6.6 and 1.4Hz); 1.17 (3H, d, J=6.1Hz) p.p.m.

## Example 8

**Potassium 5(R),3-(4-aminocarbonylphenoxy)-6(S)-[1(R)-hydroxyethyl]-7-oxo-4-thia-1-azabicyclo[3.2.0]hept-2-ene-2-carboxylate**

A mixture of 4-nitrobenxyl 5(R),3-(4-aminocarbonylphenoxy)-6S-[1(R)-hydroxyethyl]-7-oxo-4-thia-1-azabicyclo[3.2.0]hept-2-ene-2-carboxylate (5 g), dioxane (50 ml), potassium bicarbonate (1.03 g), water (50 ml) and 10% palladium on charcoal (5 g) was hydrogenolysed at 345 KPa (50 psi) for 60 minutes, was filtered through Hyflo and evaporated to half volume. The mixture was then washed with diethyl ether, and lyophilised to afford the title compound (4.0 g).

$\delta$ ($D_2O$) 7.33 and 7.87 (4H, AA'BB', J=8.8Hz); 5.74 (1H, d, J=1.3Hz); 4.28 (1H, m); 3.98 (1H, dd, J=1.3 and 6Hz); 1.32 (3H, d, J=6Hz) p.p.m.

Example 9

4-Nitrobenzyl 3-methyl-2-{4(R)-|4-(pentafluorophenoxycarbonyl)phenoxy-thiocarbonylthio|-3(S)-|1(R)-(tert-butyldimethylsilyloxy)ethyl|-2-oxoazetidin-1-yl}-2-butenoate

A solution of 4-(pentafluorophenoxycarbonyl)phenyl chlorothionoformate (1.08 g) in dry acetonitrile (5 ml) was added to a stirred solution of silver (I) 3(S)-[1(R)-(tert-butyldimethylsilyloxy)ethyl]-1-[1'-isopropylidene -1'-(4-nitrobenzyloxycarbonyl)methyl]-2-oxoazetidinone-4-thiolate (1.55 g) in acetonitrile (25 ml) and the mixture stirred as pyridine (0.103 ml) was added. When the reaction was complete as judged by t.l.c., the mixture was filtered to remove precipitated silver salts and the filtrate evaporated to leave a brown gum. Chromatography of the gum on silica gel using hexane/ethyl acetate mixtures as eluant gave the product as a pale yellow oil (1.73 g).

$\nu$ max ($CHCl_3$) 1775 (sh), 1762 (s), 1724 (m), 1522 (s), 1252 (s), 1050 (s), 910 (s) $cm^{-1}$

$\delta$ ($CDCl_3$) 8.40-8.15 (4H, m); 7.60-7.15 (4H, m); 5.97 (1H, d, J=2.7Hz); 5.25 (2H, s); 4.26 (1H, m); 3.36 (1H, dd, J=2.7Hz and 6.3Hz); 2.32 (3H, s); 2.26 (3H, s); 1.33 (3H, d, J=6.2Hz); 0.87 (9H, s); 0.22, 0.18 (6H, 2xs) p.p.m.

Example 10

4-Nitrobenzyl 2-{4(R)-[4-aminocarbonylphenoxythiocarbonylthio]-3(S)-[1(R)-(tert-butyldimethylsilyloxy)ethyl]-2-oxoazetidin-1-yl}-3-methyl-2-butenoate

A solution of 4-nitrobenzyl 3-methyl-2-{4(R)-[4-(pentafluorophenoxycarbonyl)phenoxythiocarbonylthio]-3(S)-[1(R)-(tert-butyldimethylsilyloxy)ethyl]-2-oxoazetidin-1-yl}-2-butenoate (1.73 g) in acetonitrile (20 ml) was stirred at room temperature and a solution of ammonia (0.042 g) in ethanol (1.11 ml) added. Stirring was continued for 1 hr. when solvents were removed by evaporation *in vacuo* and the residue chromatographed on 10% deactivated silica gel using hexane/ethyl acetate mixtures as eluant to afford the title compound as a yellow gum (0.84 g).

δ ($CDCl_3$) 8.17, 7.50 (4H, AA'BB', J=8.7Hz); 7.87, 7.05 (4H, AA'BB', J=8.7Hz); 6.10, 5.80 (2H, 2xbr.s); 5.97 (1H, d, J=2.7Hz); 5.22 (2H,s); 4.35-4.20 (1H,m) 3.33 (1H, dd, J=2.5 and 6.1Hz); 2.25 (3H,s); 2.04 (3H,s); 1.33 (3H, d, J=6.2Hz); 0.86 (9H,s); 0.07, 0.01 (6H,2xs) p.p.m.

Example 11

4-Nitrobenzyl 2-{4(R)-[4-aminocarbonylphenoxy-thiocarbonylthio]-3(S)-[1(R)-(tert-butyldimethylsilyl-oxy)ethyl]-2-oxoazetidin-1-yl}-2-oxoacetate

A solution of 4-nitrobenzyl 2-{4(R)-[4-aminocarbonylphenoxy-thiocarbonylthio]-3(S)-[1(R)-(tert-butyldimethylsilyloxy)ethyl]-2-oxazetidin-1-yl}-3-methyl-2-butenoate (0.84 g) in acetone (15 ml) was cooled to -70°C and added to a stirred solution of ozone (1.5 equivs) in acetone (62 ml) also at -70°C. The pale blue solution was stirred at -70°C for 30 mins. and dimethyl sulphide (1 ml) added and the mixture stirred whilst being allowed to warm up to room temperature. Volatile solvents were removed by evaporation *in vacuo* and the residue taken up in ethyl acetate (50 ml), washed with water (2 x 30 ml) and brine (20 ml). Evaporation of the dried ($MgSO_4$ anhyd.) solution gave the title compound as a colourless foam (0.68 g) contaminated with *ca*. 25% of the 4-aminocarbonylphenoxy-carbonylthio-isomer.

δ ($CDCl_3$) 8.23, 7.58 (4H, AA'BB', J=8.8Hz); 7.90, 7.25 (4H, AA'BB', J=8.6Hz); 6.30 (1H, d, J=3.7Hz); 6.06, 5.75 (2H, 2xbr.s); 5.42 (2H,s); 4.47-4.32 (1H,m); 3.62 (1H, dd, J=3.7Hz and 6.2Hz); 1.29 (3H, d, J=6.3 Hz); 0.87 (9H,s); 0.08, 0.01 (6H, 2xs) p.p.m.

Example 12

4-Nitrobenzyl 5(R),3-(4-aminocarbonylphenoxy)-6(S)-[1(R)-tert-butyldimethylsilyloxyethyl]-7-oxo-4-thia-1-azabicyclo[3.2.0]hept-2-ene-2-carboxylate

A solution of 4-nitrobenzyl 2-{4(R)-[4-aminocarbonylphenoxy-thiocarbonylthio]-3(S)-[1(R)-(tert-butyldimethylsilyloxy)ethyl]-2-oxoazetidin-1-yl}-2-oxoacetate (0.68 g) in dry chloroform (40 ml) was gently refluxed under an atmosphere of nitrogen and protected from atmospheric moisture. A solution of triethyl phosphite (0.36 ml) in chloroform (10 ml) was added dropwise *via* a syringe pump over 75 min. to the gently refluxing solution. The mixture was heated for a further 30 mins. when it was cooled and solvent removed by evaporation *in vacuo*. The crude product was chromatographed on silica gel using hexane/ether mixtures as eluant to give the title compound as a pale yellow foam (0.26 g).

δ ($CDCl_3$) 8.17, 7.55 (4H, AA'BB', J=8.7Hz); 7.83, 7.21 (4H, AA'BB' J=8.8Hz); 6.05, 5.73 (2H, 2xbr.s); 5.65 (1H, d, J=1.4Hz); 5.38, 5.21 (2H, AB, J=13.8Hz); 4.34-4.14 (1H,m); 3.76 (1H, dd, J=1.4Hz and 4.8Hz); 1.25 (3H, d, J=6.2Hz); 0.88 (9H, s); 0.09, 0.05, (6H, 2xs) p.p.m.

Example 13

4-Nitrobenzyl 5(R),3-(4-aminocarbonylphenoxy)-6(S)-[1(R)-hydroxyethyl]-7-oxo-4-thia-1-azabicyclo[3.2.0]-hept-2-ene-2-carboxylate

To a stirred solution of 4-nitrobenzyl 5(R),3-(4-aminocarbonylphenoxy)-6(S)-[1(R)-(tert-butyldimethylsilyloxy)ethyl]-7-oxo-4-thia-1-azabicyclo[3.2.0]-hept-2-ene-2-carboxylate (0.26 g) in tetrahydrofuran (3 ml) was added acetic acid (0.25 ml) followed by a 1 molar solution of tetra-n-butylammonium fluoride in tetrahydrofuran (1.31 ml). The solution was stirred at room temperature for 20 hr., diluted with ethyl acetate and the organic solution washed successively with saturated sodium bicarbonate solution, water and brine. Evaporation of the dried (anhyd. magnesium sulphate) solution left a residue which was chromatographed on 10% deactivated silica gel using hexane/ethyl acetate mixtures as eluant to give the title compound as a pale yellow solid (0.112 g).

$\nu$ max (Nujol) 3450(m), 3200(br.m), 1748(s), 1706(s), 1658(s), 1624(m) $cm^{-1}$

$\delta$ (DMSO-$d_6$) 8.18, 7.58 (4H, AA'BB', J=8.7Hz); 7.92, 7.35 (4H, AA'BB', J=8.8Hz); 8.02 (1H, br.s); 7.43 (1H, br.s); 5.78 (1H, d, J=1.4Hz); 5.40, 5.27 (2H, AB, J=14.3 Hz); 5.25 (1H, br.s); 4.04 (1H, br.m); 3.90 (1H, dd, J=6.6Hz and 1.4 Hz); 1.17 (3H, d, J=6.1Hz) p.p.m.

Example 14

4-Nitrobenzyl 5(R),3-(4-aminocarbonylphenoxy)-6(S)-[1(R)-hydroxyethyl]-7-oxo-4-thia-1-azabicyclo[3.2.0]hept-2-ene-2-carboxylate

Di-isopropylethylamine (0.101 ml) was added to a solution of diphenyl chlorophosphate (0.121 ml) and 4-nitrobenzyl 5(R),6(S)-[1(R)-hydroxyethyl]-7-oxo-4-thia-3-thioxo-1-azabicyclo[3.2.0]heptane-2-carboxylate (0.201 g) in dry acetonitrile (6 ml) and the solution stirred at $0^{o}C$ for 1½ hr. Following this a solution of 4-aminocarbonylphenol (0.08 g) in acetonitrile (1 ml) and di-isopropylethylamine (0.101 ml) were added and the mixture continued to be stirred during the time it took to reach room temperature and then at $40^{o}C$ until the reaction was complete. The mixture was partitioned between ethyl acetate and water and the organic layer washed with water, dried over anhydrous magnesium sulphate and evaporated in vacuo. Chromatography of the residue on 10% deactivated silica gel using hexane/ethyl acetate mixtures as eluant gave the title compound (0.026 g).

The spectral data of this product was identical to that of the compound prepared in Example 13.

Example 15

O-(4-Aminocarbonylphenyl) carbonochloridothioate

To a vigorously stirred mixture of thiophosgene (5 ml) and water (10 ml) at 0° was added dropwise a mixture of 4-hydroxybenzamide (3 g) water (30 ml) and 2M sodium hydroxide (14.2 ml). The precipitate was filtered and washed with water, with dichloromethane, and then dried in vacuo to afford the title compound (4.08 g).

δ (acetone-$d_6$) 8.10 and 7.42 (4H, AA'BB', J=7.9Hz); 7.62 (1H, broad) and 6.85 (1H, broad) p.p.m.

Example 16

4-Nitrobenzyl 3-(4-aminocarbonylphenoxy)-2-{3(S)-[1(R)-(tert-butyldimethylsilyloxy)ethyl]-4(R)-propythio-azetidin-2-on-1-yl}-3-trimethylacetyl-thiopropenoate

To a stirred mixture of 4-nitrobenzyl 2-{3(S)-[1(R)-(tert-butyldimethylsilyloxy)ethyl]-4(R)-propylthioazetidin-2-on-1-yl}acetate (2 g) in dry tetrahydrofuran (12 ml) was added at -40° a preformed mixture of hexamethyldisilazane (2 ml), dry tetrahydrofuran (6 ml) and 1.55M-n-butyllithium in hexane (5.8 ml). After 15 minutes a solution of O-(4-amino carbonylphenyl) carbonochloridothioate (0.955 g) in dry tetrahydrofuran (6 ml) was added, followed by trimethylacetyl bromide (1.07 ml). After

15 minutes the mixture was poured onto a mixture of diethyl ether and 2M-hydrochloric acid. The organic layer was separated; the aqueous layer was washed with ethyl acetate, and the combined organic layers were washed with water, with saturated potassium bicarbonate, with water and with brine, and were evaporated in vacuo. Chromatography of the residue on silica gel afforded the title compound (1.04 g) as a yellow solid as a mixture of the E- and Z- isomers.

δ ($CDCl_3$) 8.23 and 8.07 (2H, 2xd, J=8.7Hz); 7.76 (3H,m); 7.58 and 7.35 (2H, 2xd, J=8.7Hz); 7.09 (3H,m); 5.30 (3H,m); 4.23 (1H,m) 3.20 (1H,dd, J=2.9 and 3.9Hz); 2.61 (2H,m); 1.34 (3H, d, J=6Hz); 1.3 (2H, m); 1.10 and 1.03 (9H, 2xs); 0.96-0.79 (12H,m); 0.09 and 0.06 (3H, 2xs); 0.01 (3H,s) p.p.m.

Example 17

4-Nitrobenzyl 3-(4-aminocarbonylphenoxy)-2-{3(S)-[1(R)-(tert-butyldimethylsilyloxy)ethyl]-4(R)-chloro-azetidin-2-on-1-yl}-3-trimethylacetylthiopropenoate

A solution of chlorine (43 mg) in carbon tetrachloride (0.51 ml) was added to a stirred mixture of 4-nitrobenzyl 3-(4-aminocarbonylphenoxy)-2-{3(S)-[1(R)-(tert-butyldimethylsilyloxy)-ethyl-4(R)-propylthio-azetidin-2-on-1-yl}-3-trimethylacetylthiopropenoate (0.33 g), propylene oxide (0.0015 ml) and chloroform (5 ml) at -45°. The mixture was evaporated to dryness to afford the crude title compound (0.33 g). Chromatography of a portion

over silica gel and evaporation _in vacuo_ afforded a sample of the pure title compound as a mixture of E- and Z- isomers.

δ ($CDCl_3$) 8.23 and 8.10 (2H, 2xd, J=8.8Hz); 7.79 (3H,m); 7.56, 7.37 (2H, 2xd, J=8.8Hz); 7.08 (2H,m); 6.08 and 6.05 (1H, d, J=1.5Hz); 5.30 (2H,m); 4.24 (1H,m); 3.47 (1H, dd, J=1.5 and 3.4 Hz); 1.24 (3H, d, J=7Hz); 1.10 and 1.03 (9H, 2xs); 0.86, 0.79 (9H, 2xs); 0.08, 0.04, 0.01 (6H, 3xs).

## Example 18

4-Nitrobenzyl 5(S),3-(4-aminocarbonylphenoxy)-6(S)-[1(R)-(tert-butyldimethylsilyloxy)ethyl]-7-oxo-4-thia-1-azabicyclo[3.2.0]hept-2-ene-2-carboxylate

Pyridine (0.038 ml) was added to a stirred mixture of 4-nitrobenzyl 3-(4-aminocarbonylphenoxy)-2-{3(S)-[1(R)-(tert-butyldimethylsilyloxy)ethyl]-4(R)-chloro-azetidin-2-on-1-yl}-3-trimethylacetylthiopropenoate (132 mg), tetrahydrofuran (2 ml) and water (0.2 ml). After two hours the mixture was evaporated _in vacuo_ and chromatographed on silica gel. Elution with hexane - ethyl acetate mixtures afforded the title compound (66 mg) with physical properties identical to those described in Example 5.

CLAIMS

1. A process for the production of a compound of formula Ia

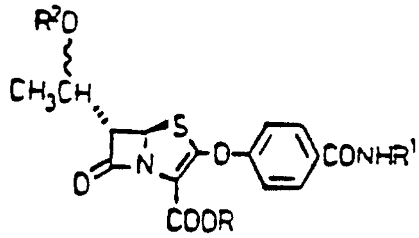

(Ia)

in which R represents a hydrogen atom or a carboxy protecting group,

$R^1$ represents a hydrogen atom or an alkyl group, having from 1 to 4 carbon atoms,

and

$R^2$ represents a hydrogen atom or a hydroxy protecting group,

or a salt thereof, which comprises

(1) producing a compound of formula Ib

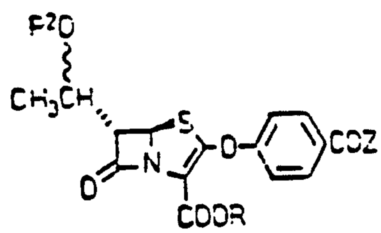

(Ib)

in which R and $R^2$ are as defined above, and Z represents an , $-NHR^3$, $-NR^3R^3$, $-NR^1R^3$, $-R^4$ or -OH group in which

$R^3$ represents a nitrogen protecting group (in the case of $-NR^3R^3$ the two groups $R^3$ may be the same or different), and

$R^4$ represents a carboxylic acid activating group, or the group $XR^{5'}$ with X being oxygen or sulphur and $R^{5'}$ being phenyl, substituted by 1 - 5 substituents selected from the group consisting of CN, $CH_3O$, fluorine, chlorine, or

-COZ represents -CN, by

(A) heating a compound of formula II

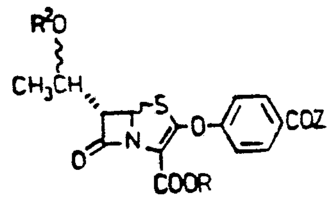

(II)

in which R, $R^2$ and Z are as defined above, or

(B) reacting a compound of formula IV

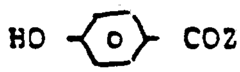

(IV)

in which Z is as defined above, optionally in the presence of a base with a compound of formula III

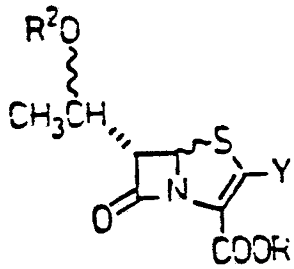

(III)

in which R and $R^2$ are defined as above, and Y represents a leaving group selected from groups of formula

$-X\overset{\overset{O}{\|}}{P}(OR_a)_2$ or $-X\overset{\overset{O}{\|}}{P}(R_a)_2$

in which X represents an oxygen or sulphur atom and the two groups $R_a$, which may be the same or different, each represents a straight or branched chain alkyl group having from 1 to 6 carbon atoms, or a phenyl group which may be unsubstituted or substituted by one or more substituents selected from halogen atoms, cyano, nitro and methyl groups,

groups $-OSO_2R_b$ in which $R_b$ represents a straight or branched chain alkyl group having from 1 to 4 carbon atoms, which may be substituted by one or more fluorine atoms, or $R_b$ represents a phenyl group that is unsubstituted or is substituted by a p-nitro, p-bromo- or p-methyl group;

groups $-\overset{O}{\uparrow}{S}R_a$ and $-SO_2R_a$ in which $R_a$ is defined as above; and;

groups

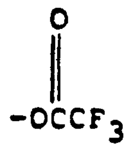

and if the resulting compound has the formula II

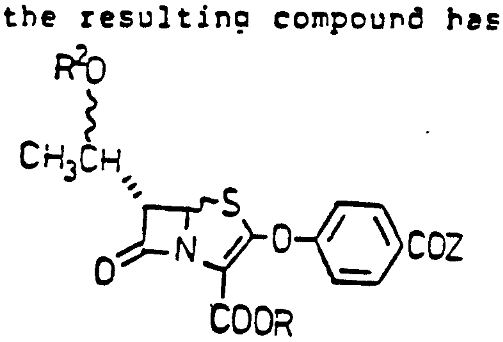

(II)

in which R, $R^2$ and Z are defined as above, converting compound II to compound Ib and/or isolating compound Ib from compound II,

(C) cyclising a compound of formula Va, Vb or Vc

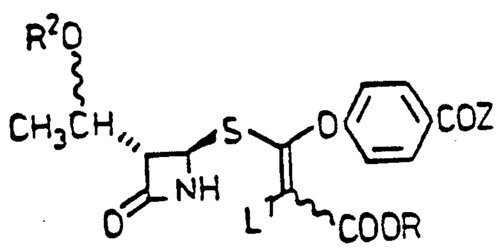

(Va)

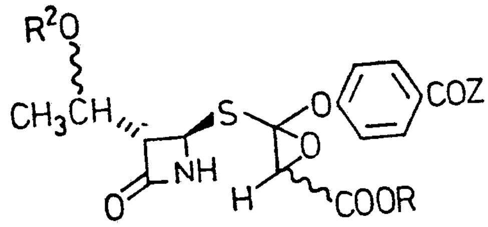

(Vb)

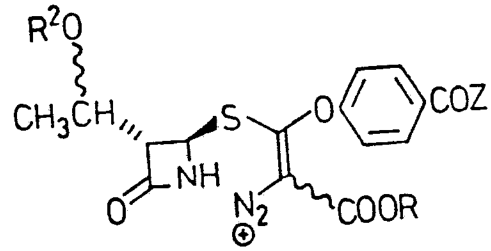

(Vc)

in which R, $R^2$ and Z are as defined above, and $L^1$ represents a chlorine or bromine atom,

(i) in the case of compound Va eliminating $HL^1$

(a) by treatment with a base and/or a metal or a metal-containing reagent,

(b) by electrolysis,

(c) by photolysis, or

(d) by reaction with a free radical-producing compound optionally in the presence of an initiator;

(ii) in the case of compound Vb by treatment with a Lewis acid or a base, and

(iii) in the case of compound Vc by treatment with a rhodium- or copper-containing compound;

(D) cyclising a compound of formula VIa, VIb, VIc or VId,

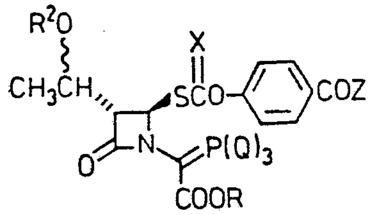

(VIa)

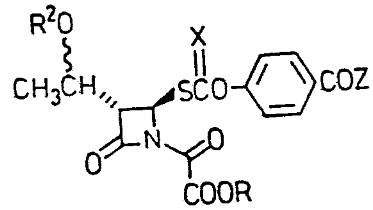

(VIb)

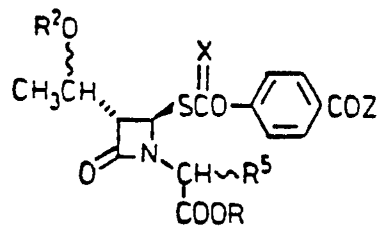

(VIc)

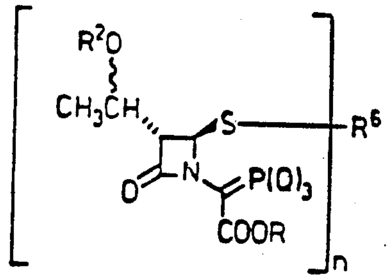

(VId)

in which formulae R, $R^2$ and Z are defined above, X represents an oxygen or sulphur atom, the group $=P(Q)_3$ represents a group derived from a trivalent organophosphorus reagent, $R^5$ represents a bromine or chlorine atom, $R^6$ repesents Cu(II), Pb(II) or Hg(II), in which case n represents 2, or represents Ag(I), in which case n represents 1,

(i) in the case of compound VIa, by heating compound VIa or allowing compound VIa to stand at room temperature;

(ii) in the case of compound VIb, by treatment with a trivalent oganophosphorus reagent and heating the resulting compound or allowing the resulting compound to stand at room temperature;

(iii) in the case of compound VIc, by treatment with a phosphine and a base, and then heating the resulting compound or allowing the resulting compound to stand at room temperature,

(iv) in the case of compound VId by reaction with a compound of formula VII

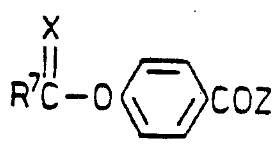

(VII)

in which X and Z are as defined above and $R^7$ represents an activating group, for example, an activating ester group or a halogen atom, and heating the resulting compound or allowing the resulting compound to stand at room temperature or below;

(E) cyclising a compound of formula VIIIa or VIIIb

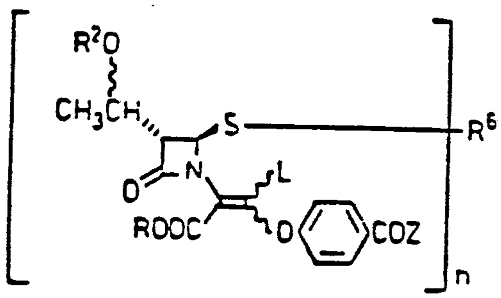

(VIIIa)

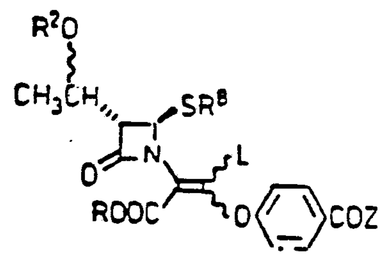

(VIIIb)

in which formulae R, $R^2$, $R^6$, n, and Z are defined as above and

L represents a leaving group that can be replaced in a nucleophilic displacement reaction,

$R^8$ represents a hydrogen atom or a sulphur protecting group,

(i) in the case of compound VIIIa, effecting cyclisation, and

(ii) in the case of compound VIIIb, removing the sulphur protecting group, if present, and either reacting the resulting thiol compound with a reagent capable of converting the compound into compound VIIIa, and cyclising compound VIIIa, or cyclising the thiol compound by treatment with a base, and

carrying out any of one or more of the reactions set out in the further steps (2) to (4) in any appropriate order:

(2) in a resulting compound in which -COZ represents other than a $-CONHR^1$ group carrying out one of the following reactions,

(a) when -COZ is $CONHR^3$ , $CONR^1R^3$ or $CONR^3R^3$, removing the protecting group(s) $R^3$;

(b) when -COZ is $COR^4$, reacting the compound with ammonia or methylamine, or a chemical equivalent thereof;

(c) when -COZ is -COOH, activating the -COOH group, and then reacting the resulting compound with ammonia or a lower alkylamine;

(d) when -COZ represents -CN, converting the group -CN into the group $-CONHR^1$;

(3) in a resulting compound of formula Ia, Ib or II in which the free or protected 8-hydroxy group has S-stereochemistry carrying out a stereochemical inversion to give the corresponding 8R compound;

(4) if desired or required, carrying out any one or more of the following steps in any desired order:

a) hydrolysing a 2-carboxylic ester group in a compound to give the corresponding free acid,

b) treating a free acid compound or a salt thereof with an agent capable of forming a 2-carboxylic acid ester, for example, with an alcohol, a phenol or a reactive derivative thereof, to give a 2-carboxylic acid ester thereof,

c) carrying out an acid- or a base-catalysed ester interchange on a 2-carboxylic acid ester to give a different ester of that compound,

d) treating a free acid compound with a base to give a salt at the carboxy group at position 2,

e) treating a 2-carboxylic acid ester having a basic group present with an acid to give an acid addition salt thereof.

f) treating a salt of a compound with an acid to give a free acid of that compound,

g) removing a hydroxy protecting group from a compound having a protected 8-hydroxy group to give the corresponding compound having a free 8-hydroxy group,

h) treating a compound having a free hydroxy group at the 8-position with an organic acid derivative to form an ester at the 8-position.

2. A process as claimed in claim 1(A), wherein the compound of formula II has the following structure IIa

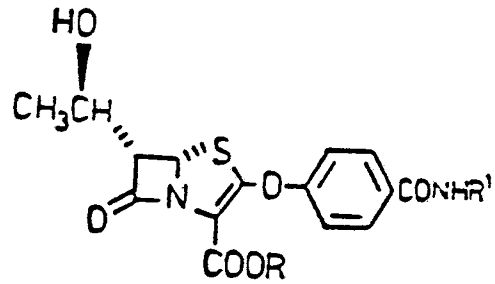

(IIa)

in which R represents a carboxy protecting group and $R^1$ is as defined in claim 1.

3. A process as claimed in claim 1(C), wherein a compound of formula Va is produced

a) by reacting a compound of formula XIX

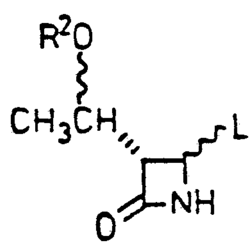

(XIX)

in which $R^2$ and L are as defined in claim 1, with a compound of formula XXIb

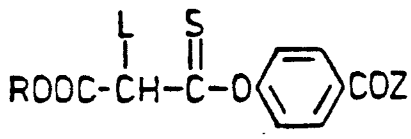

(XXIb)

in which R, L and Z are as defined in claim 1, that has been treated with a base, or

b) by reacting a compound of formula XX

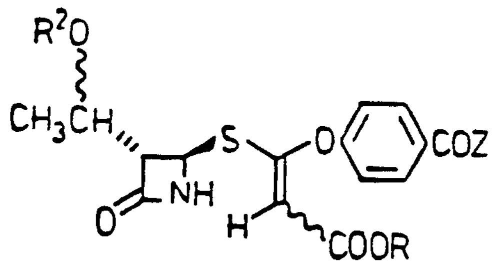

(XX)

in which R, $R^2$ and Z are defined as in claim 1, with a halogenating agent.

4. A process as claimed in claim 1(C), wherein a compound of formula Vb is produced by reacting a compound of formula XX as defined in claim 3, with hydrogen peroxide optionally in the presence of a base, or with a peracid.

5. A process as claimed in claim 1(C), wherein a compound of formula Vc is produced by carrying out a diazo transfer on a compound of formula XX as defined in claim 3.

6 . A process as claimed in claim 3, wherein a compound of formula XX is produced by reacting a compound of formula XIX as defined in claim 3 with a compound of formula XXIa

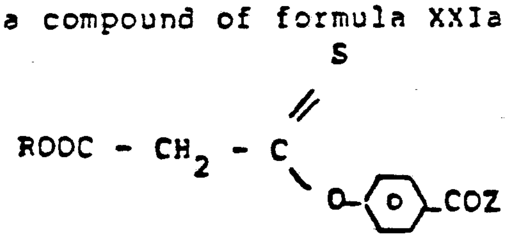

(XXIa)

in which R and Z are defined as in claim 1.

7 . A process as claimed in claim 1(D), wherein a compound of formula VIb is produced by reacting a compound of formula XXII

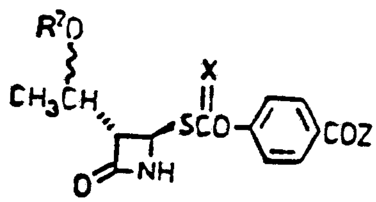

(XXII)

in which R, $R^2$, X and Z are as defined in claim 1, with a compound of formula XXV

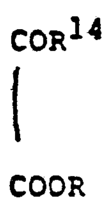

(XXV)

in which R is as defined in claim 1, and $R^{14}$ represents a group that can be displaced by the azetidinone nitrogen in the compound of formula XXII.

8 . A process as claimed in claim 7, wherein a compound of formula XXII is produced by reacting a compound of formula XIX

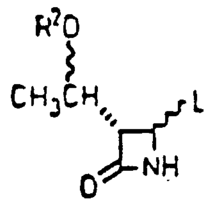

(XIX)

in which $R^2$ and L are defined as in claim 1 with a compound of formula XXIII

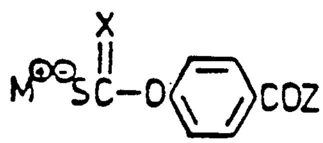

in which X and Z are defined as in claim 1, and M represents an alkali metal or alkaline earth metal atom, or an ammonium group that is unsubstituted or substituted by one to four groups selected from alkyl groups having from 1 to 4 carbon atoms.

European Patent Office

# EUROPEAN SEARCH REPORT

0 257 419

EP 87 11 1529

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | EP-A-0 174 561 (HOECHST)<br>* Claims *<br>--- | 1-8 | C 07 D 499/00 //<br>C 07 F 7/18<br>C 07 D 205/08 |
| D,Y | EP-A-0 170 028 (HOECHST)<br>* Claims * & GB-A-2 161 161<br>--- | 1-8 | |
| A | EP-A-0 069 377 (HOECHST)<br>* Claims *<br>--- | 1-8 | |
| A | GB-A-2 042 515 (BRISTOL-MYERS)<br>* Claims *<br>----- | 1-8 | |
| | | | **TECHNICAL FIELDS SEARCHED (Int. Cl.4)** |
| | | | C 07 D 499/00 |

The present search report has been drawn up for all claims

| Place of search | Date of completion of the search | Examiner |
|---|---|---|
| THE HAGUE | 16-11-1987 | CHOULY J. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document